# EUROPEAN PATENT SPECIFICATION

(11) **EP 4 308 924 B1**
(45) Date of publication and mention of the grant of the patent: **07.05.2025**
(21) Application number: 22711598.7
(22) Date of filing: 16.03.2022
(51) Int. Cl.: G01N 33/50, B01L 3/00, C12Q 1/6881

(54) **METHOD OF SELECTING CELLS**
VERFAHREN ZUR AUSWAHL VON ZELLEN
MÉTHODE DE SÉLECTION DES CELLULES

(30) Priority: 16.03.2021 GB 202103609
(43) Date of publication of application: 24.01.2024
(60) Divisional application: 25158050.2
(73) Proprietor: Lightcast Discovery Ltd, Cambridge CB3 0FA (GB)
(72) Inventor: ISAAC, Thomas Henry, Cambridge Cambridgeshire CB3 0FA (GB); HEAD, Emma, Cambridge Cambridgeshire CB3 0FA (GB); SOSNA, Maciej, Cambridge Cambridgeshire CB3 0FA (GB); DIECKMANN, Nele Marie Godette, Cambridge Cambridgeshire CB3 0FA (GB); MURZEAU, Claire Marie, Cambridge Cambridgeshire CB3 0FA (GB); ATHANASOPOULOU, Evangelia-Nefeli, Cambridge Cambridgeshire CB3 0FA (GB); WAEBER, Andreas, Cambridge Cambridgeshire CB3 0FA (GB); FRAYLING, Cameron, Cambridge Cambridgeshire CB3 0FA (GB); LOEFFEN, Paul, Cambridge Cambridgeshire CB3 0FA (GB)
(74) Representative: Stratagem IPM Limited
(86) International application number: PCT/GB2022/050671
(87) International publication number: WO 2022/195281

(56) References cited:
- EP-A1- 3 656 473
- WO-A1-2020/104769
- US-A1- 2009 203 063

## Description

### FIELD OF THE INVENTION

The present invention relates to a method using a device for selecting cells, on the basis of desirable characteristics. These selected cells can then be expanded for therapeutic or manufacturing use. The methods may be useful in adoptive cell therapy (ACT), notably cellular immunotherapy, but also has other uses, particularly in the characterisation of cells from a population, commonly where the cells may be genetically engineered. Further, the method may be used to select cells based upon their ability to produce a desired molecule, such as an immunoglobulin or immunotherapeutic drug. Synthetic biology is a rapidly developing field, and may utilise cells to produce pharmaceutical drugs, fuels, foods and beverages, or to produce enzymes that may degrade plastics and other pollutants. Devices of the present invention are in the field of microfluidics and relate to microfluidic devices capable of manipulating microdroplets.

### BACKGROUND OF THE INVENTION

The rapid and high-throughput processing of cells is a significant challenge during research, development and manufacturing. This is particularly true concerning the generation of biotherapeutics such as cell therapies and cell-derived therapeutics, where identification and selection of cells is required, whilst retaining the cell for further expansion or testing. Existing methods for sorting, assaying and selecting cells remain slow and cumbersome, despite the development of microfluidic devices.

The approval of cell-based therapies is increasing, and it is paramount to ensure that the cells infused into patients have desirable characteristics, and furthermore do not have characteristics that could lead to any risk to the patient. The use of cells removed from a patient, genetically engineered (gene therapy) and replaced into the patient for many diseases is known, including for inherited disorders of the immune system, hemoglobinopathies, metabolic diseases, and cancer. Other uses of cell-based gene therapy are known in diseases and disorders of the eye.

In one example, genetic engineering equips T cells with a cell surface receptor known as a chimeric antigen receptor (CAR). Such CAR-T cells have a key advantage in being able to bind to cancer cells via expressed cell surface markers and provoke a specific anti-tumour response. Whilst currently licensed therapies involve autologous re-infusion of T cells, the use of allogenic T cells in the future is possible. Nevertheless, it is of great importance only to use CAR-T cells that have a stable phenotype, and that are expressing the correct CAR on their surface, without modification, and that this CAR does not display "on target/off-tumour" and indeed "off target" binding ability, such that healthy cells of the patient are targeted. Additionally, it is useful to determine that the CAR-T has efficacy against its target prior to infusion. The two currently approved CAR-T cell therapies have numerous known deficiencies with regards to dose consistency, patient safety, and manufacturing process impacts. It is understood that these deficiencies are shared throughout the fields of adoptive cell therapy and gene therapy.

Important underlying factors include:
- Extreme variations in yield between treatments and manufactured doses, giving unpredictable efficacy in patients.
- Known off-target effects (such as CNS off-targets and cardiac targets) give severe adverse response in some patients.
- Variable yields from leukapheresis give a requirement to maintain maximum cell viability and careful selection of input cell sub-populations is required.
- Contaminants (such as B-cells) can spoil the culture
- It is difficult or impossible to predict patient responses as there are no straightforward automated ways to measure T-cell activity against a tumour target.

Further, cells can be employed to make or manufacture products for numerous uses, including therapeutic indications, assays, research, food and beverages, agriculture, and as raw materials. This effectively uses the cells to produce products that may be harvested from the cells as a secreted product, such as immunoglobulins, or harvested by lysing the cells to recover intracellular products such as drug molecules.

Of particular interest is the manufacture of immunoglobulins from hybridomas and lymphocytes. It is often desirable to identify a cell producing an immunoglobulin with particular antigen-binding capabilities, such that this cell may be expanded or analysed if particular antigen-binding characteristics are determined. Such immunoglobulins are usually antibodies, such as monoclonal antibodies and may be used therapeutically or for assays and research. In selecting a cell or cells with such characteristics, it may be necessary to screen many thousands of cells. Since the intermediate steps between harvesting and identification of cells producing a desired product can cause losses and/or reduced performance, it is best to load cells directly onto device and reduce handling steps. Without prior FACs sorting, the ratio of desirable cells to undesirable cells is greatly reduced. Here a large droplet handling and screening capability is required.

Selecting cells for desirable characteristics of producing a product can result in the cell being damaged or destroyed during that testing, which in undesirable when selecting cells which make a desirable product.

There is thus a need for a robust method that streamlines the labour intensive process of screening cells for desirable characteristics, permits selection and later expansion or further testing of such cells, rejection of specific cells with undesirable characteristics, and reduces overall time and cost. Thus, it is important that not only the cell can be tested but also recovered. Microfluidic platforms have been proposed for solving this need, however as yet no methods have been proven to be practical and reliable in a clinical setting. In particular, the ability to carry out simultaneous multiplexed assays on a plurality of cells has proven elusive, and furthermore the ability to process many cells simultaneously, in a high-throughput format. Indeed, much of the work on such devices is determined by the use of fixed structures, such as sequestration pens, which does not permit the flexibility to perform multiple assays on cells. Several platforms do not permit the investigation at the individual cell level, instead performing assays on a plurality of cells. Further, inflexible systems mean that it is possible only to run a single assay on the cell, as there are no mechanisms to permit successive assays to take place, or to retain the cell for further expansion once the assays are complete. Miniaturisation of assays reduces the volumes of reagents consumed during assaying steps and so lowers the costs of consumable materials.

The present disclosure provides methods for cell selection in which cells are placed into microdroplets and wherein the actuation mechanism for manipulating the cell-containing microdroplets on the surface of a microfluidic chip, is electrowetting on dielectric (EWOD), optionally optically mediated electrowetting on dielectric (oEWOD).

The use of such chip based devices advantageously allows for the manipulation of microdroplets across a wide range of sizes, and being digitally controlled, provides for dynamically re-programmable operational steps. This device structure permits more elaborate and integrated workflows compared to conventional approaches, such as independent control of the microdroplets, as well as allowing for a greater density of microdroplets to be controlled across regions of the microfluidic chip surface.

### SUMMARY OF THE INVENTION

The present disclosure provides methods wherein the flexibility of EWOD or oEWOD microfluidic chips is utilised to enable high throughput processing of microdroplets, thus solving the need for multiplexed handling in screening applications. A method for selecting cells as claimed herein comprises cell(s), part(s) of cell(s) and/or cell derived species as disclosed herein.

Provided herein are methods of selecting cells, particularly selecting cells for a therapeutic uses or manufacturing, the methods of the disclosure are useful, *inter alia,* in adoptive cell therapy, gene therapy and cell based manufacturing. The cells selected may be a subset of a larger population of cells, wherein the cells are generally of the same type or same species. The cells selected may not be of the same type or same species. The cells may be selected so as to provide heterogeneity within a cell population. The cells could be genetically the same but could have certain genes switched on or off.

Provided herein are methods for selecting cells for investigating cell interactions with other entities, such as (bio)chemical/(bio)molecule entities, or other cells (cell-cell interactions). For example, the methods may comprise targeted cell-cell interactions where two or more selected cells are brought together.

Provided herein are methods for selecting cell(s) for investigating secretion(s) from single cell(s) or cell-cell interactions. For example, the methods may comprise targeted cell-cell interactions where two or more selected cells are brought together.

According to one aspect of the disclosure, there is provided a method for selecting cells in an EWOD or oEWOD device, the method comprising:
i. providing a test panel of microdroplets, comprising at least a medium or a medium and at least one cell;
ii. providing at least one reporter panel of microdroplets, containing one or more reporter entities;
iii. merging microdroplets of the test panel with microdroplets of the at least one reporter panel to form a panel of merged assay microdroplets; and
iv. monitoring the panel of assay microdroplets using a detection system capable of detecting a change in said reporter entity based upon the presence of at least one characteristic, and
v. selecting a subset of microdroplets from a panel on the basis of the change in the reporter entity, said subset containing the selected cells;

wherein the test panel of microdroplets comprising at least medium is created by splitting a panel of microdroplets containing at least one cell and a medium, further creating a reference panel of microdroplets containing at least one cell;
and/or wherein the test panel of microdroplets comprising at least one cell are split into at least two panels of microdroplets before or after any of steps (i) to (v), said panels being: a test panel of microdroplets comprising at least medium or medium and at least one cell, suitable for merging with microdroplets of a reporter panel; and a reference panel of microdroplets containing at least one cell.

The cells may therefore be selected based upon one or more characteristics.

The methods described herein may involve at least one splitting operation, for example such that the panel of microdroplets is split initially to provide the test panel of microdroplets containing at least a medium and the reference panel of microdroplets containing at least one cell, prior to step (i).

If the panel of microdroplets in step (i) contains one or more cells, it is not necessary to have undergone a splitting operation before step (iii).

Alternatively or additionally one or more further splitting operations may be included at any appropriate step in the method. The split may produce test panels of microdroplets containing at least a medium, and/or a test panel of microdroplets containing at least a medium and at least one cell.

The splitting operation may be performed such that if the cell is capable of producing secretions, that any secretions are included in the medium. The splitting step may be performed such that multiple child droplets are created which may have differing concentrations of said secretion. Alternatively or additionally, splitting operations can be performed at differing times, such that the child microdroplets may contain differing concentrations of secretions.

The splitting operations may result in the formation of one or more reference panel of microdroplets containing at least one cell, and one or more test panels of microdroplets, which may contain a medium with or without at least one cell.

The methods can conceivably include steps involving both test panels of microdroplets including at least one cell and microdroplets containing at least medium, depending on the characteristics of interest.

The aim of such splitting operations is to generate a reference panel of microdroplets, containing at least one cell.

Alternatively, there is provided a method for selecting cells in an EWOD or oEWOD device, the method comprising:
i. providing a panel of microdroplets, comprising one or more cells and a medium;
ii. splitting said panel of microdroplets into at least two panels of microdroplets, at least one reference panel of microdroplets comprising one or more cells and at least one test panel of microdroplets comprising at least said medium;
iii. providing at least one reporter panel of microdroplets, containing one or more reporter entities;
iv. merging microdroplets of the test panel with microdroplets of the at least one reporter panel to form a panel of merged assay microdroplets; and
v. monitoring the panel of assay microdroplets using a detection system capable of detecting a change in said reporter entity based upon the presence of at least one characteristic, and
vi. selecting a subset of microdroplets from the reference panel on the basis of the change in the reporter entity in the corresponding assay microdroplet panel, said subset containing the selected cells.

The methods of the present disclosure involve the detection or determination of one or more characteristics of a cell. As used herein, the characteristic of a cell may be any suitable characteristics, such as any one or more of: the presence of one or more cell surface molecules; the presence of one or more cellular activities; cellular morphology; the presence of one or more cellular secretions; and/or the presence of one or more intracellular products.

The characteristics of the cell may be determined according to any suitable means according to the methods described herein. The method may use one or more reporter entities that are capable of determining or detecting the presence of a characteristic. Such reporter entities may be provided within a microdroplet, for merging with the microdroplet which is undergoing testing for said characteristic.

The methods as described herein may relate to the selection of cells, wherein a panel of microdroplets containing at least one cell are split, such that a panel of microdroplets containing at least one cell is generated, such is defined here as the reference panel. This splitting step or splitting operation may be conducted at any appropriate point or step in the method, such that the reference panel is constructed at an appropriate point. This reference panel may be constructed at different points/steps in the method depending upon the cells under selection. The reference panel may therefore contain a sub-set of the cells originally under consideration.

The splitting step also results in the generation of at least one test panel of microdroplets. The test panel of microdroplets may contain medium from the progenitor microdroplet. Should the cell be capable of secretion, the medium may also contain secretions from said cell. The test panel and the reference panel are correlated such that results of interrogation of the test panel and selection of desirable characteristics may result in selection of the corresponding reference microdroplet containing at least one cell. Thus, the results from interrogation of the test panel may be correlated to the progenitor cell and the same is selected.

Prior to the splitting step, the panel of microdroplets containing at least one cell may be cultured under conditions permitting cell division, optionally clonal expansion. Such conditions may include providing additional medium to the cells via merging a panel of microdroplets containing medium with the panel of microdroplets containing at least one cell. Other conditions may include the provision of supplements such as growth factors, nutrients, cell signalling molecules, chemicals and the like to the cells via merging a panel of microdroplets containing supplemented medium with the panel of microdroplets containing at least one cell.

If the cells in the panel of microdroplets are permitted to divide, the reference panel and the test panel of microdroplets may be split such that the reference panel or microdroplets contains at least one clonal copy of a cell corresponding to a cell contained in the test panel of microdroplets. Thus, the results from interrogation of the test panel may be correlated to the progenitor cell and the same is selected.

According to one aspect, the test panel of microdroplets containing at least one cell may be merged with a panel of microdroplets containing an agent for lysing said cells. Such a step may be required if the characteristic of the cell to be determined is the presence of an intracellular product. Alternatively, an intracellular product may be capable of direct detection using a reporter entity that can enter the cell.

Thus, the cell in the microdroplets that form the panel may be permitted to undergo cell division prior to exposure to particular reporter entities. Once the cell has undergone division, each microdroplet in the panel can be split, such that at least two child microdroplet panels are formed, and the microdroplets therein comprising at least one cell. At least one microdroplet panel can be reserved as a reference panel, and at least one other panel can be used in the method of the invention ("test panel"). The reference panel and the test panel therefore contain corresponding or correlating cells, and results for the test panel can result in identification of its corresponding cell in the reference panel. It may be described that the test panel of microdroplets and the reference panel of microdroplets each contain cells derived from the original progenitor cell, and therefore are genetically identical or are clones.

According to any aspect of the disclosure, cells in any panel of microdroplets containing at least one cell may be inspected for their morphological characteristics and said morphological characteristics may be used to select a subset of cells. This inspection may occur before step (ii) of the method, or may occur before or after any step of the method. The morphological characteristics may include size, shape, adhesion state, cell membrane features such as blebs and and/or the presence of intracellular features such as vacuoles.

Selection of cells according to any aspect means defining a sub-set of cells, and discarding or discounting the remaining cells. Selection may be positive (selection of a cell based upon the presence of a characteristic) or negative (selection of a cell based upon absence of a negative characteristic). The discarded/discounted microdroplets may be dispensed from the device or retained within the panel and discounted from consideration.

According to some aspects, prior to step (i) the panel of microdroplets containing at least one cell may be subjected to at least one assay to determine a characteristic of the cell, said assay being performed by merging said panel of microdroplets containing at least one cell with at least one panel of microdroplets containing at least one reporter entity and determining a change in the at least one reporter entity based upon the presence of said characteristic. Thus, prior to any splitting step, initial inspections or interrogations may be performed.

According to one aspect, once selected, the cell may be dispensed from the device, either for expansion or for further testing.

According to another aspect of the disclosure, there is provided a method of selecting cells using an EWOD or oEWOD device comprising the steps of:
i) providing a panel of microdroplets, comprising at least one cell and medium;
ii) providing at least one panel of reporter microdroplets, comprising one or more reporter entities;
iii) merging microdroplets of the panel comprising at least one cell with microdroplets of the at least one reporter panel to form a merged panel of microdroplets;
iv) monitoring the merged panel of microdroplets using a detection system capable of detecting a change in said reporter entity based upon one or more characteristics of the cell and selecting a subset of microdroplets on the basis of said change in a reporter entity to form a selected panel of microdroplets comprising a cell;
v) taking the selected panel of microdroplets comprising a cell and optionally repeating steps (ii) to (iv) thereon one or more times, in which one or more different reporter entities are used in step (ii);
vi) selecting cells based upon the final panel of microdroplets selected in step (v);
wherein prior to step (iii) and/or step (v) the panel of microdroplets comprising at least one cell are split into at least two panels of microdroplets, , said panels being:
a. a test panel of microdroplets, comprising at least medium, suitable for merging with microdroplets of a reporter entity panel; and
b. a reference panel of microdroplets comprising at least one cell.

In one aspect, prior to splitting the panel of microdroplets containing at least one cell, the cells may be cultured to permit cell division, such that the test panel of microdroplet may contain a cell, optionally a clonal cell copy of the cell from the reference panel.

Alternatively, according to one aspect, the present invention provides:
a method of selecting cells using an EWOD or oEWOD device comprising the steps of:
   i) providing a first panel of microdroplets comprising at least one cell;
   ii) providing at least one panel of reporter microdroplets containing one or more reporter entities;
   iii) merging microdroplets of the first panel with microdroplets of the at least one reporter panel to form a first panel of merged assay microdroplets;
   iv) monitoring the panel of assay microdroplets using a detection system capable of detecting a change in said reporter entity based upon one or more characteristics of the cell and selecting a first subset of microdroplets on the basis of said change in a reporter entity to form a first selected panel of microdroplets;
   v) providing at least one further reporter panel of microdroplets, containing one or more reporter entities;
   vi) merging microdroplets from the selected panel of microdroplets with microdroplets from the further reporter panel of microdroplets to form a second panel of merged assay microdroplets;
   vii) monitoring the second panel of assay microdroplets using a detection system capable of detecting a change in said reporter entity based upon one or more characteristics of the cell and selecting a second subset of microdroplets on the basis of said change in a reporter entity to form a second selected panel of microdroplets; said subset containing the selected cells;
wherein prior to step (iii) and/or step (v) the panel of microdroplets comprising a cell are split into at least two panels of microdroplets, said panels being:
   a. a panel of microdroplets, comprising at least medium, suitable for merging with microdroplets of a reporter panel; and
   b. a reference panel of microdroplets, comprising at least one cell.

The following features may apply to any variant of the methods disclosed herein:
Splitting of microdroplets may be equal or unequal, such that a microdroplet can be split into 2 or more microdroplets, the 2 or more microdroplets each being the same or different sizes.

In one aspect, prior to splitting the panel of microdroplets containing at least one cell, the cells may be cultured to permit cell division, such that the test panel of microdroplet may contain a cell, optionally a clonal cell copy of the cell from the reference panel.

According to an embodiment of the methods disclosed herein, the panel of microdroplets in step (i) may contain at least one cell, or may contain more than one cell if the original cell has undergone cell division. The aim of the methods of the invention is to characterise a specific cell type, wherein the characteristics of the cell over the whole population of cells of the same type to be assayed may be variable. The method may include the step of disposing or discarding microdroplets that contain a plurality of different cells of the same type, which have not arisen due to cell division.

According to an embodiment of the methods disclosed herein, the panel of microdroplets in step (i) may contain at least medium. Said microdroplets containing at least a medium have previously been divided or split from microdroplets containing a cell in said medium. In advance of the splitting step, the panel of microdroplets containing at least one cell may be merged with a panel of microdroplets containing medium, which may optionally include signalling molecules or cells to encourage the cell to produce secretions. The medium in the test panel of microdroplets may therefore contain a secretion from the cell, which is to be exposed to reporter entities in order to define a characteristic of the cell. The method may include the step of disposing or discarding microdroplets that do not contain a secretion of interest. The droplets containing medium may be of differing volume to the droplets containing cells.

Further, optionally, the reporter panel used for the selection of cells permit identification of cells with desirable characteristics, for example cell surface characteristics such as the expression of a specific cell surface marker or secretion of a desirable molecule. A reporter entity in such rounds may be an entity that binds or interacts with a cell surface molecule or secreted molecule.

Optionally a selection step permits identification of cells with desirable cell:cell interaction characteristics, for example cell binding, activation, cell fusion, cellular uptake or cell death. A reporter entity in such rounds of selection may be a cell such as a tumour cell.

Optionally a selection step may include assays to look at viability, activation and/or expansion characteristics or ability.

Optionally the cell in the test panel of microdroplets, if present, is lysed to release cell contents. Such a step may be considered if the cell has already been subjected to a cell division event, and a reference panel of microdroplets, each comprising at least once cell, has been created.

Microdroplets can be removed from the panel at any point during the method if it is determined that no further analysis is required. For example, microdroplets may be discarded if the cell does not include desirable characteristics, does not undergo cell division, and does not possess the desirable characteristic, such as promoting cell death in a reporter entity cell or release an antibody capable of binding to an antigen.

It may be desirable to analyse any kind of cell using the methods of the present invention, but the cells may be of the same type, for example they are B cells or T cells (lymphocytes), and the methods of the invention may be used to select cells with a particular characteristic, which may be inherently present in said cell, or may be genetically engineered in said cell. The cell is also described as a biological cell. It may be desirable to analyse co-cultures where the cells within the droplets are of disparate types such as a combination of reporter cells and primary cells, or a culture combining epithelial cells of different phenotypes to form a tissue-like structure. The cell(s) may be natural. The cell(s) may be artificial. The cell(s) may be microcells. The methods of the invention may be cell free and use part(s) of a cell(s), for example nuclei and/or mitochondria. The methods of the invention may use liposomes.

The methods of the invention may be used to select cells with desirable characteristics for use in therapy.

The methods of the invention may be used to select cells with desirable characteristics for use in research.

The methods of the invention may be used to select cells with desirable characteristics for use in the manufacture of a desirable product, such as a molecule/compound/chemical. In such uses, the cells may be genetically engineered bacterial cells or the like.

### DECRIPTION OF THE FIGURES

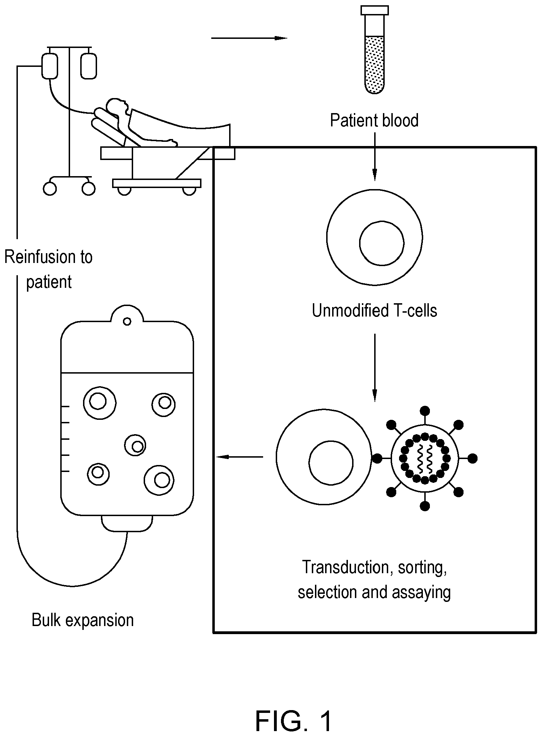

**Figure 1****:** shows an exemplary method, wherein blood from a patient is collected and T-cells are isolated, transduced, sorted and selected before bulk expansion and reinfusion into the patient. Also shown is the section within this manufacturing process targeted by our CAR-T workflow.

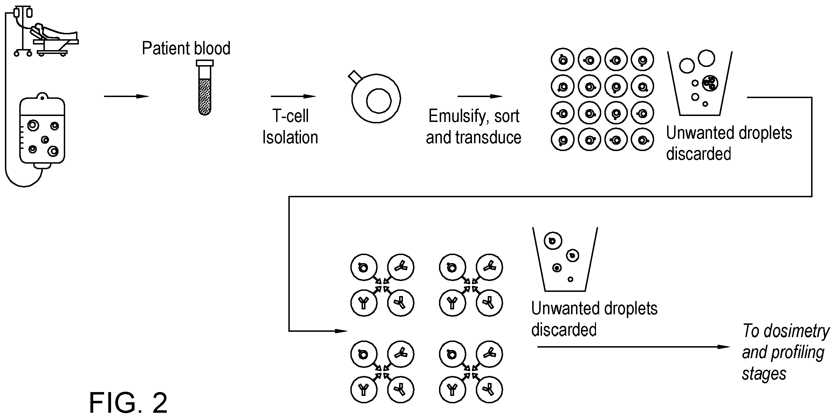

**Figure 2****:** shows an overview of the initial stages in an exemplary CAR-T workflow before dosimetry and profiling, wherein T-cells isolated from patient blood are emulsified into microdroplets, sorted and transduced. Unwanted microdroplets are discarded and a multi-reporter cell-surface marker assay is performed on the cells of the remaining microdroplets. On the basis of this assay, a panel of microdroplets are selected.

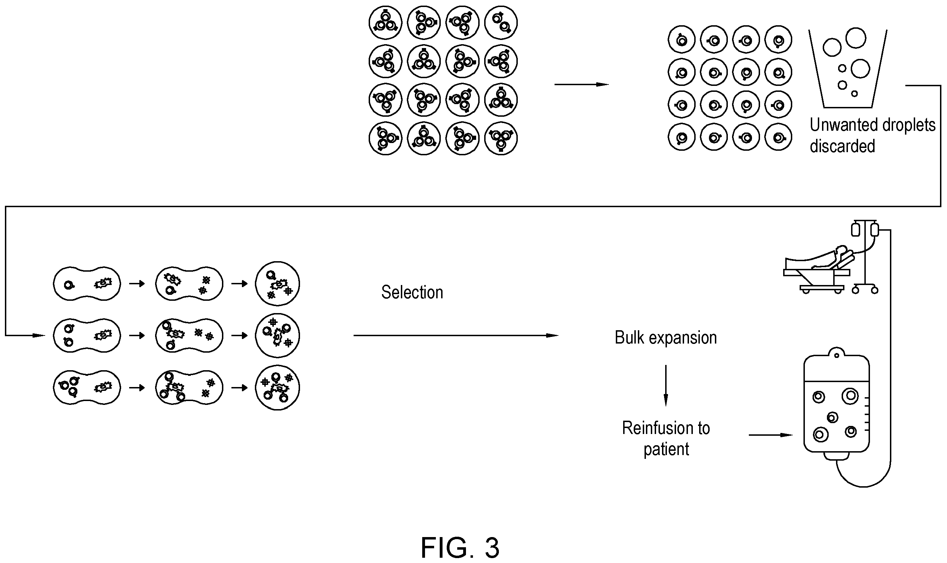

**Figure 3****:** shows an overview of the later stages in an exemplary CAR-T workflow, wherein following the multi-reporter cell-surface marker assay, cells of a selected panel of microdroplets are assessed for cell viability, activation and exhaustion behaviour. The panel of microdroplets may be split such a CAR-T cell is retained in a reference panel and a clonal CAR-T cell is used in the further selection steps. Activation assessment involves, for example, merging microdroplets containing T-cells with microdroplets containing cancer cells and quantifying cancer-cell killing activity. A final panel of microdroplets is then selected; T-cells are dispensed from the device for bulk expansion and reinfusion into a patient.

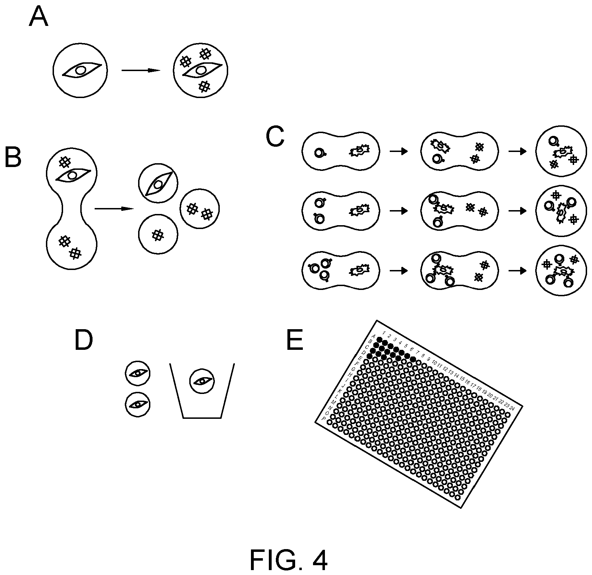

**Figure 4****:** outlines an exemplary immuno-oncology workflow wherein: A) CHO cells are modified to produce an immunotherapeutic drug and loaded into microdroplets; B) the microdroplets are split into child droplets containing equal or different doses of drug depending on the time-point of split; C) microdroplets containing T-cells are merged pairwise with microdroplets containing cancer cells in a first merge operation, the merged microdroplets are then merged with microdroplets containing the doses of drug in a second merge operation; D) positive hits are determined by quantifying cancer cell death and CHO cells corresponding to positive hits are selected; and E) selected CHO cells are dispensed into a well plate and expanded.

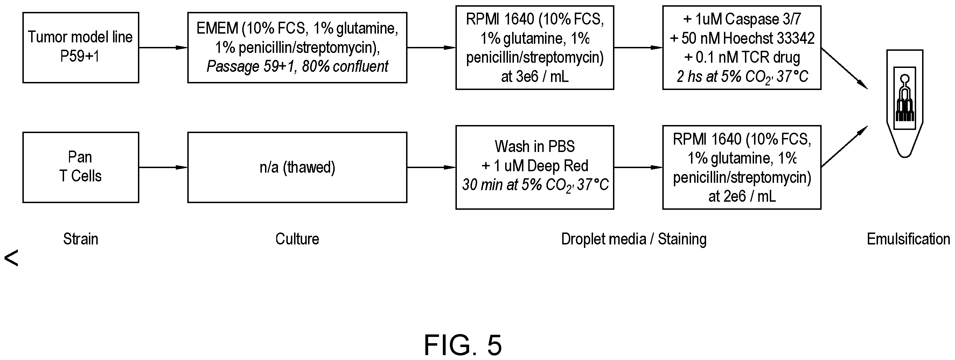

**Figure 5****:** shows an exemplary preparation protocol for generating microdroplets containing either T-cells or tumour cells and TCR drug.

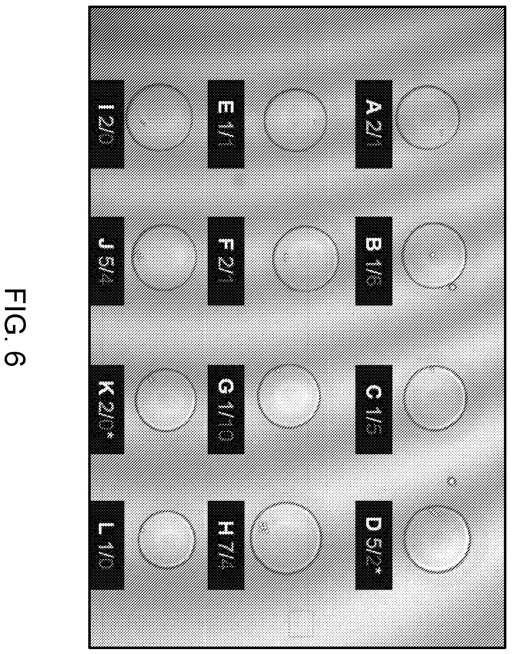

**Figure 6****:** shows a panel of microdroplets, each contain T-cell(s) and tumour cell(s) at various T-cell(s)/tumour cell(s) ratios. For each droplet, the number of tumour cell(s) is indicated in each box on the left and the number of T-cell(s) is indicated in each box on the right. Droplet I, K and L are negative controls containing only tumour cells.

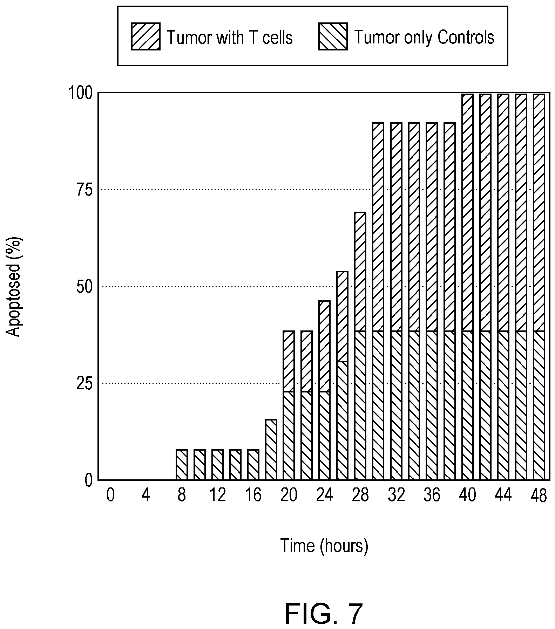

**Figure 7****:** shows a plot of time in hours versus percentage of cancer cells apoptosed when contacted with T cells. Comparisons against tumour-only controls are given. In this case the apoptosis is acting as a reporter of the anti-tumour killing activity of the T cells under the test conditions, which can be used as a selection criteria for later expansion of the T-cells or sampling them for further analysis

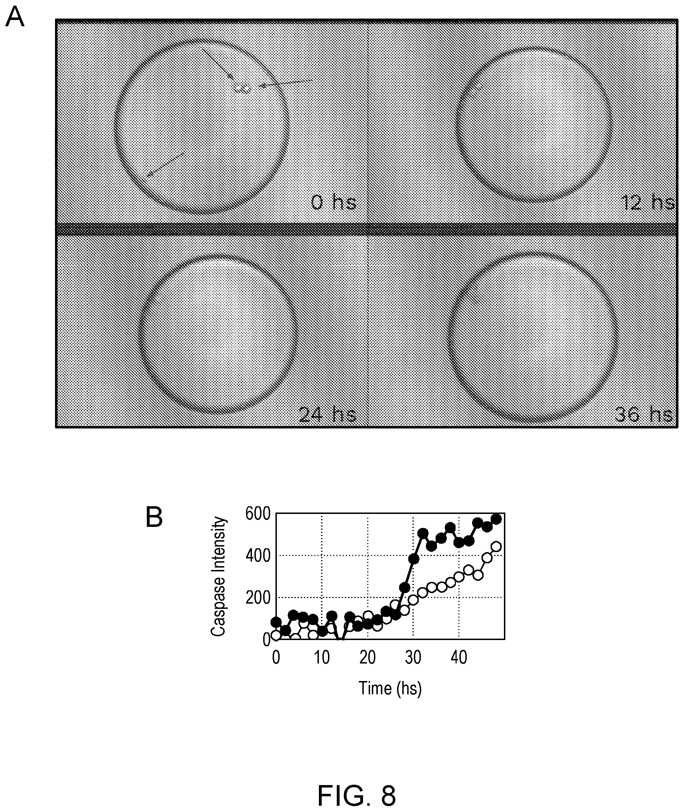

**Figure 8:** 8A shows Brightfield images of a microdroplet containing a T-cell and two tumour cells taken periodically over 36 hours. The T-cell is shown to serially kill the two tumour cells. The graph (8B) confirms this tumour cell killing activity by quantifying caspase fluorescence intensity for each tumour cell over the same timeline.

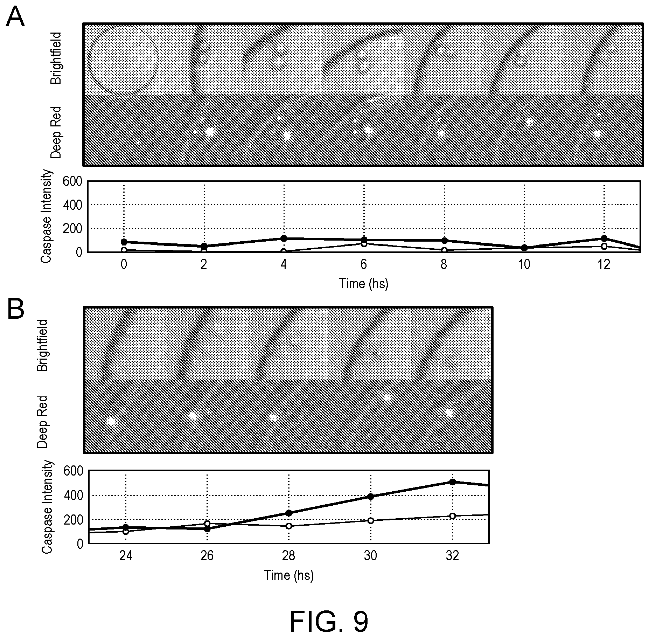

**Figure 9**: 9(A): close up Brightfield and Deep Red stained images of a microdroplet containing a T-cell and two tumour cells taken periodically over the first ~13 hours of a 36 hour experiment showing caspase fluorescence intensity quantified over the same timeline; and 9(B) close up Brightfield and Deep Red stained images of the same microdroplet taken periodically over the last ~10 hours of the experiment also showing caspase fluorescence intensity quantified over the same timeline. The serial tumour cell killing activity of the T-cell can be visualised here.

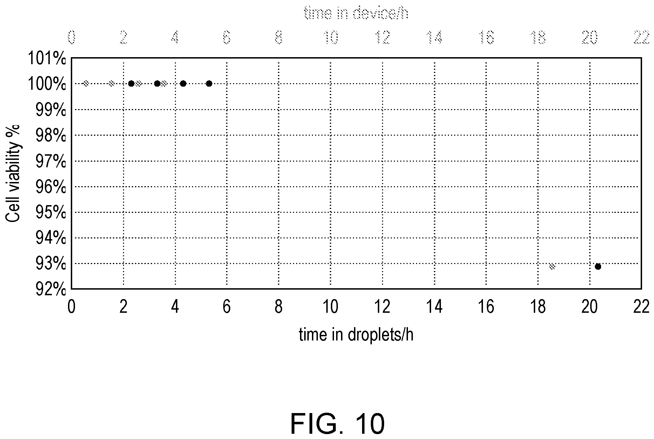

**Figure 10****:** shows hybridoma cell viability expressed as a percentage against time, wherein the cells are either in the device or in droplets. Cell viability remains at 100% for up to 5.3 hours in droplets and 3.6 hours in the device and at >90% for up to 20.3 hours in droplets and 18.6 hours in device.

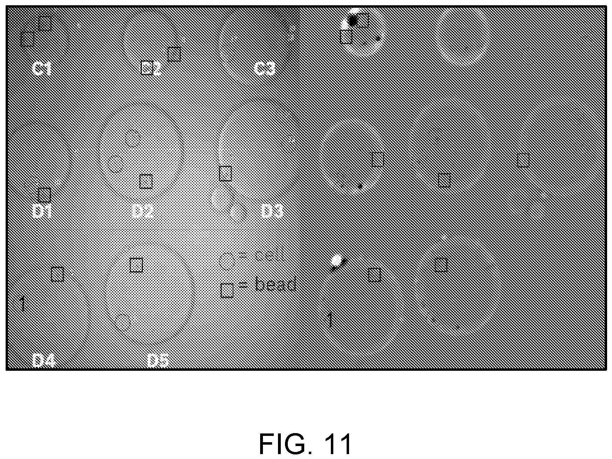

**Figure 11****:** Left) shows an image of a panel of microdroplets containing hybridoma cell(s) and bead(s) or bead(s) only or hybridoma cell(s) only. Beads are indicated by squares whilst cells are indicated with circles. C1 and C2 each contain only beads, C3 contains only cells, D1 to D3 each contain 2 cells and 1 bead, D4 and D5 each contain 1 cell and 1 bead. Right) shows an image of the same panel also showing bead fluorescence. Fluorescent aggregates appeared to varying degrees in D1 to D5. No fluorescence was detected in microdroplets containing only cells or only beads (controls).

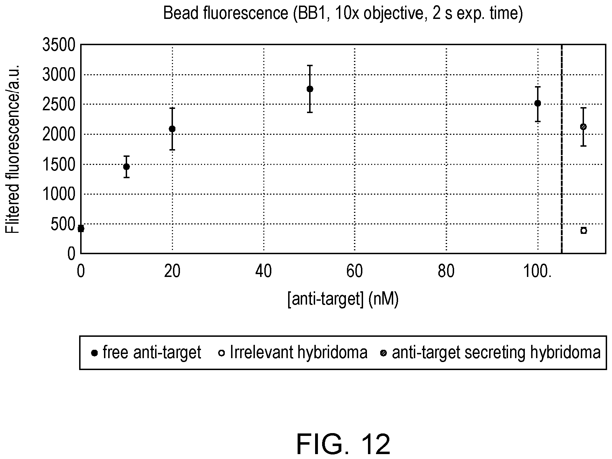

**Figure 12****:** shows a graph with filtered AF488 fluorescence on the Y axis and free anti-target antibody concentration on the X axis. On the far right hand side, average filtered fluorescence is plotted for a population of irrelevant hybridomas and a population of target hybridomas. It can be seen that fluorescence close to maximum level observed for free anti-target antibody was measured for secreting target hybridoma cells.

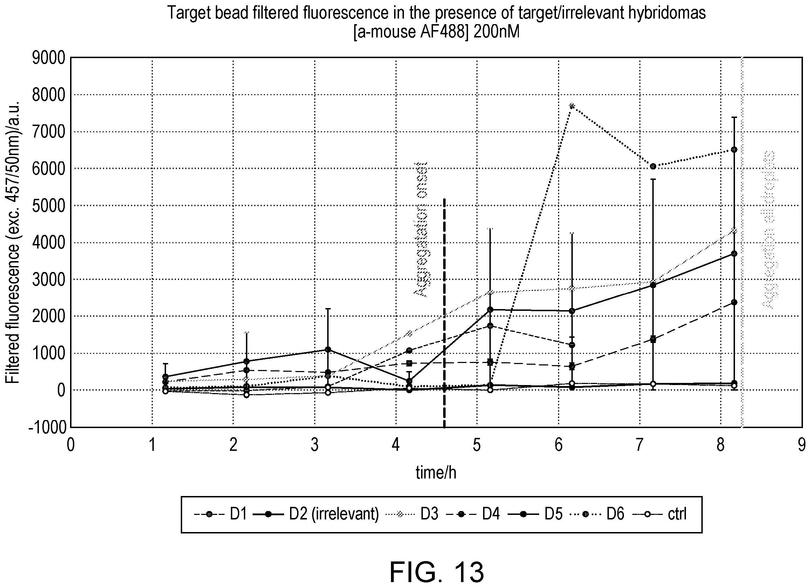

**Figure 13****:** shows a graph presenting data for a multiplex spiking assay with target bead filtered AF488 fluorescence on the Y axis and time on the X axis. Target bead filtered AF488 fluorescence is measured against time for a population of microdroplets, each containing target or irrelevant hybridoma cell(s) and reporters. Also included is a negative control. D2 which contained irrelevant hybridoma cell(s) remained dark and is indistinguishable from the negative control(s). All target hybridomas show a positive signal.

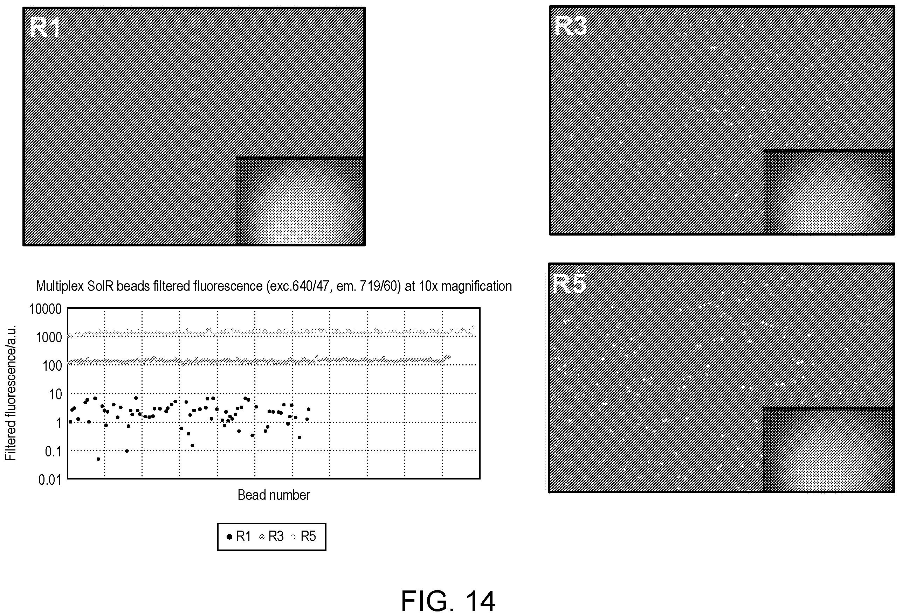

**Figure 14****:** shows fluorescence and Brightfield (inset) image of SolR1, R3, R5 beads using a 10x objective. The graph shows filtered florescence measured for a population of SolR1, R3 and R5 beads in multiple allowing beads to readily be distinguished.

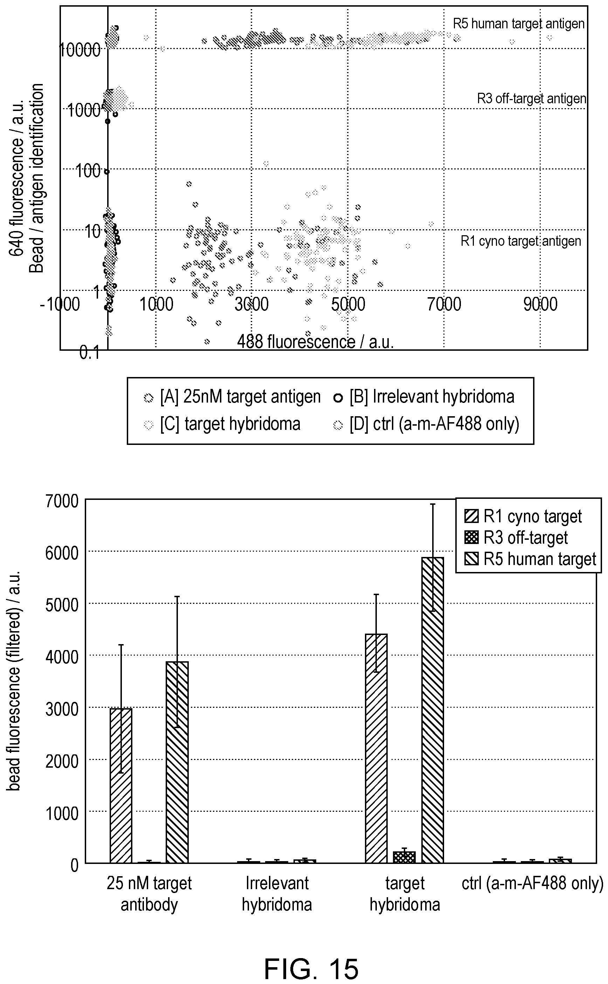

**Figure 15****:** Top) a graph presenting data for a multiplex bead assay with bead filtered fluorescence on the Y axis and AF488 filtered fluorescence on the X axis. Filtered AF488 fluorescence is measured at each bead for a population of microdroplets containing either target or irrelevant hybridomas and reporters. The beads are decoded by a detection system (R5 human target, R3 off-target and R1 cyno target). Positive (25 nM free target antibody) and negative (anti-mouse AF488 only) control microdroplets are also assayed. It can be seen that negative control microdroplets remain dark whilst positive control microdroplets confirm the binding of free target antibody to both target antigens (R1 and R5). Microdroplets containing secreted antibody also bind both target antigens. Bottom) shows a bar graph with filtered AF488 fluorescence on the Y axis and microdroplet category on the X axis where each category is further split into R1, R3 and R5 sub categories.

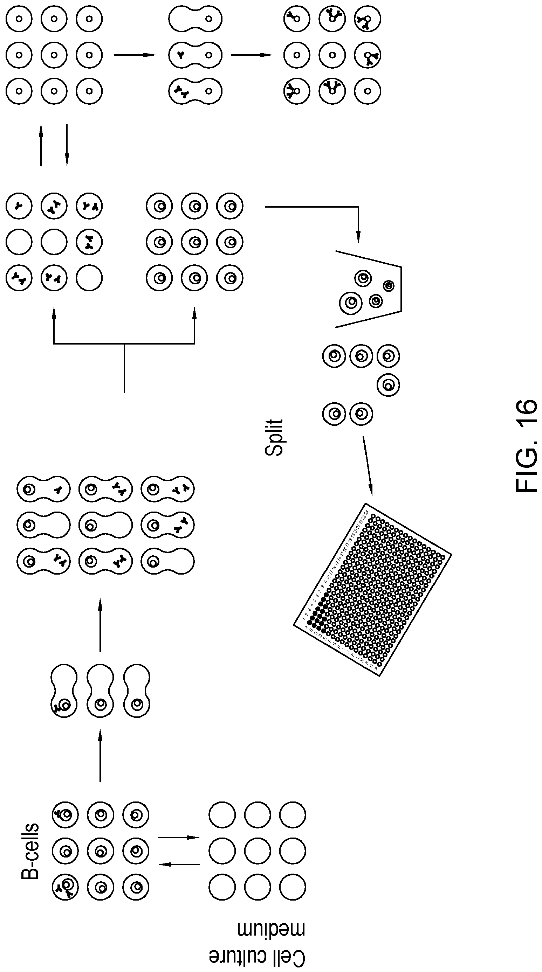

**Figure 16****:** outlines an exemplary monoclonal antibody discovery workflow wherein: B cells are loaded into microdroplets and merged with microdroplets containing cell culture media pairwise for added volume to facilitate subsequent splitting steps; the merged microdroplets are split into child droplets to obtain multiple doses of secreted antibody produced by each cell; the microdroplets containing B-cells are sorted into a reference panel whilst the microdroplets containing doses of antibody are sorted into an test panel; the test panel is merged pairwise with a reporter panel of microdroplets; an optical detection system is used to determine positive hits in the merged reporter-mAb panel; and microdroplets of the reference panel containing B-cells corresponding to said positive hits are dispensed into a well plate and expanded.

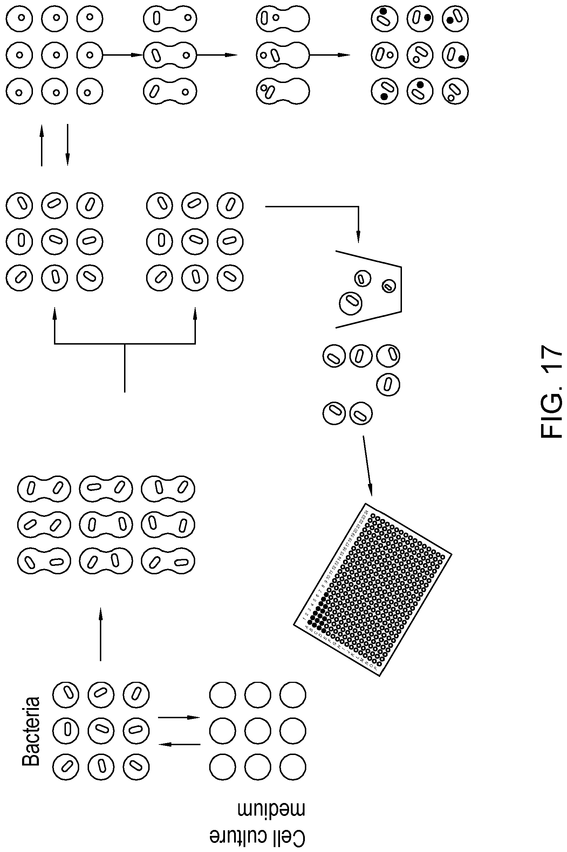

**Figure 17****:** outlines an exemplary bacterial cell workflow wherein: bacterial cells are loaded into microdroplets and merged with microdroplets containing cell culture media pairwise for added volume to facilitate subsequent splitting step; the merged microdroplets are permitted to undergo cell division and then split into clonal colonies; one set of microdroplets containing clonal bacterial cells is retained as a reference panel whilst a further set of microdroplets containing clonal bacterial cells or a set of microdroplets containing secretions of said bacterial cells is sorted into a test panel of microdroplets; the test panel of microdroplets is merged pairwise with a reporter panel of microdroplets; the bacterial cells may be lysed to release their contents; an optical detection system is used to determine positive hits in the merged reporter-bacterial cell panel; and microdroplets of the reference panel containing bacterial cells corresponding to said positive hits are dispensed into a well plate and expanded.

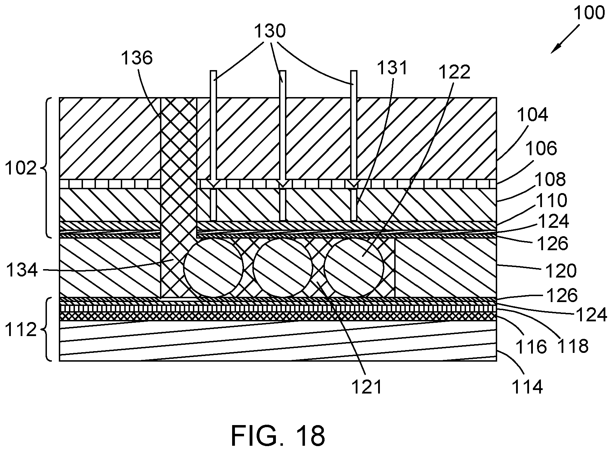

**Figure 18****:** shows an oEWOD configuration.

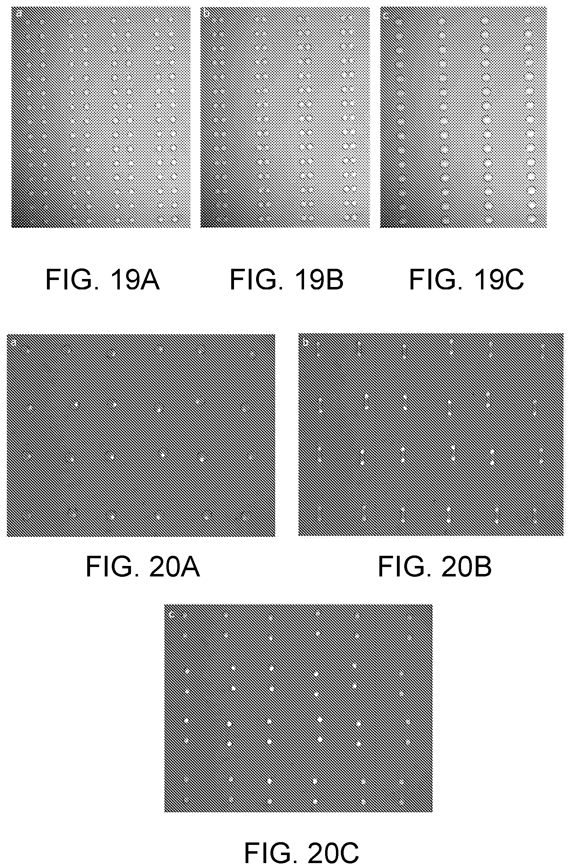

**Figure 19A** provides an image of a droplet merge operation with parallel merging of droplet pairs.
**Figure 19B** provides an image of paired microdroplets moving together prior to merging.
**Figure 19C** provides an image of merged microdroplets.
**Figure 20A** shows a droplet splitting operation with parallel splitting of droplets.
**Figure 20B** shows stretched droplets midway through a splitting operation.
**Figure 20C** shows droplets split into pairs of daughter droplets.

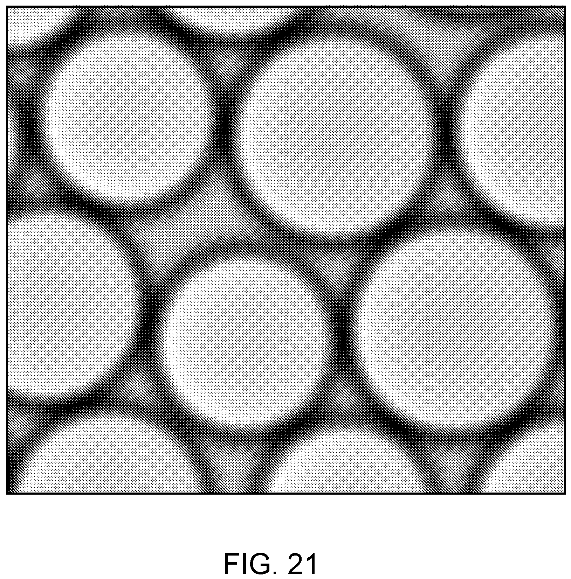

**Figure 21** provides an image of *S. cerevisiae* bio-particles (heated inactivated yeast) encapsulated in microdroplets at single cell occupancy.

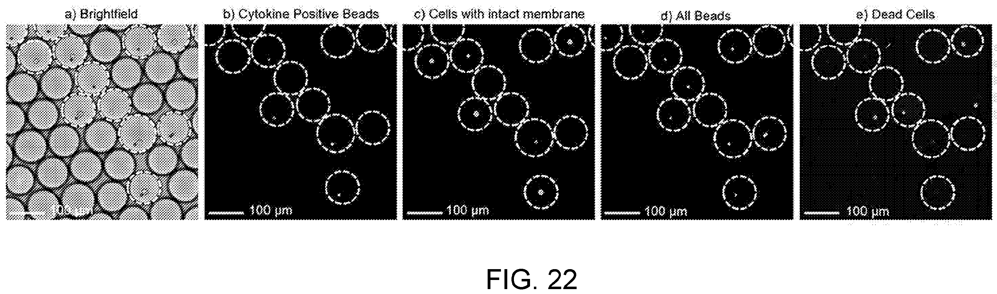

**Figure 22** provides an image of cytokine secreting cells co-encapsulated with cytokine reporter beads within the microdroplets in an emulsion of cell media in oil. In the presence of secreted cytokine the reporter beads fluoresce via a sandwich ELISA mechanism using a fluorescently tagged antibody. The brightfield image (a) shows the distribution of cells and beads within the droplets. Fluorescence images indicate b) which reporter beads give a positive response in the presence of secreted cytokines, c) the position of all cells with intact membrane integrity, d) the position of all beads, and e) dead cells as indicated by propidium iodide staining.

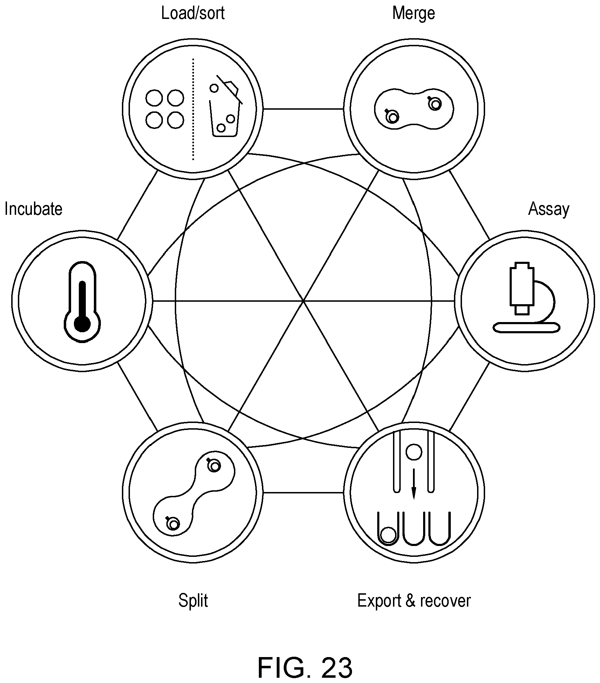

**Figure 23** is a flexible and unique workflow operation of microdroplet, including microdroplet manipulations such as loading, sorting, merging, splitting operations. Programmable decisions can be implemented at any point throughout the workflow, which can divert from the original order and/or apply operations to only a selection of droplets.

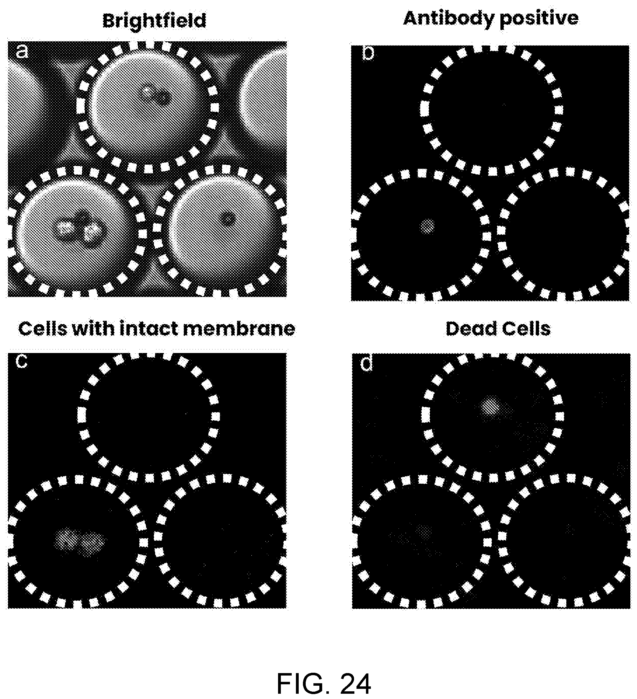

**Figure 24****:** Antibody secreting cells are co-encapsulated with reporter beads within droplets in an emulsion of cell media in oil. In the presence of secreted antibody the reporter beads fluoresce via a sandwich ELISA mechanism using a fluorescently tagged antibody. The brightfield image (a) shows the distribution of cells and beads within the droplets. Fluorescence images indicate b) which reporter beads give a positive response in the presence of secreted antibody, c) the position of all cells with intact membrane integrity, and d) dead cells.

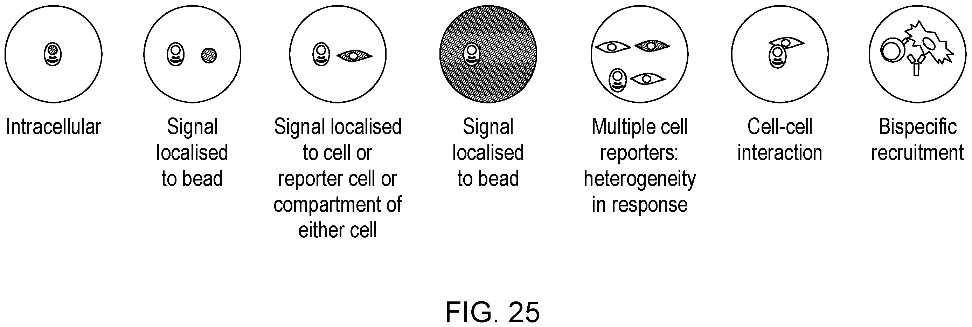

**Figure 25****:** A variety of detection modes for monitoring cell secretions or cell-cell interactions.

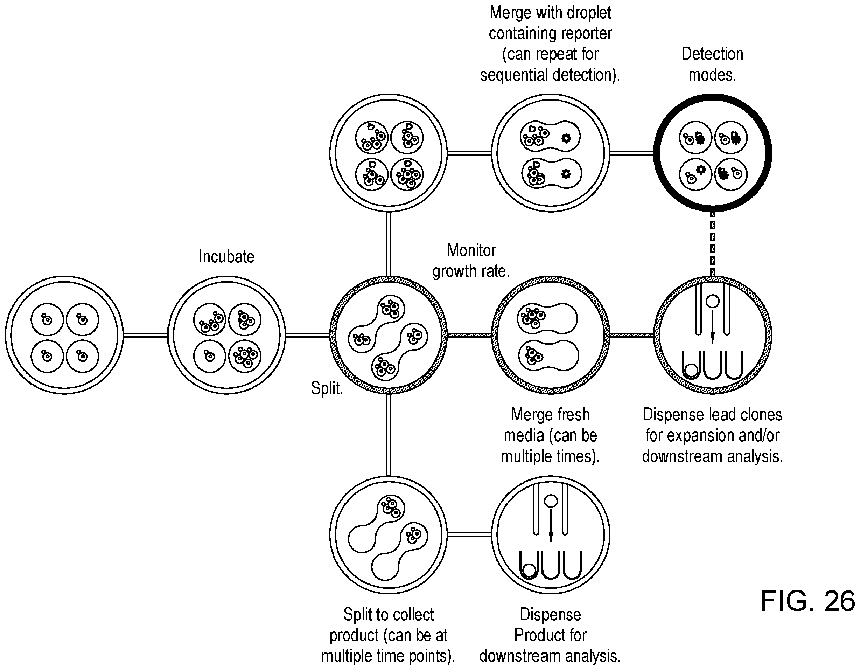

**Figure 26****:** A workflow showing a typical microdroplet operation. The microdroplets containing cells can be manipulated such as splitting, merging, sorting operations. The microdroplets can also contain reporters.

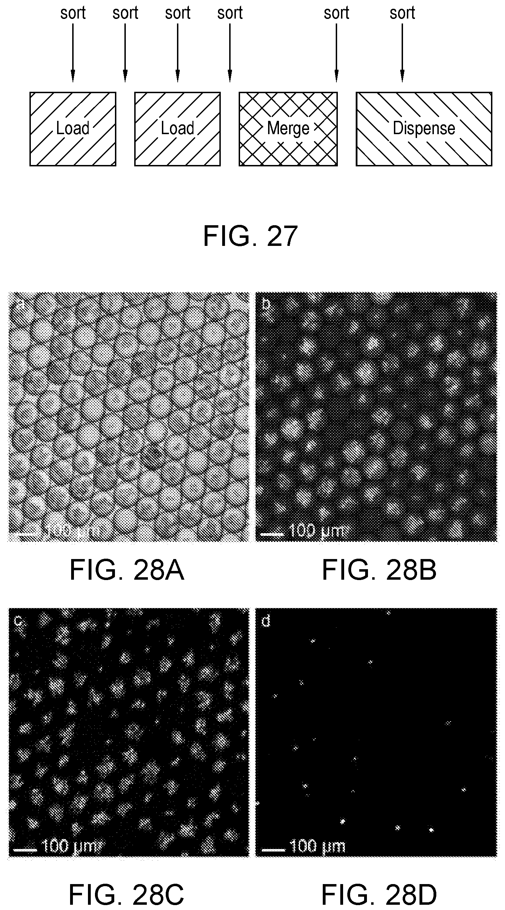

**Figure 27****:** A workflow describing sorting options and/or combination of merging, and dispensing operations.
**Figures 28A to 28D****:** Various images of cells encapsulated within the microdroplets, incubated in the presence of a dye for intracellular Calcium.

### DETAILED DESCRIPTION OF THE INVENTION

The inventors have found that the deficiencies in the prior art methods of selecting cells may be overcome, by using the methods disclosed herein. The use of the methods disclosed herein results in better product quality, predictability and a higher number of favourable cell selections.

The methods of the disclosure are unique, since they propose, *inter alia,* testing cells for a plurality of characteristics, such that the cells with the desired characteristics and best profile may be selected, and in terms of cell therapy this may be the best safety profile. The methods disclosed permit the selection of cells with the desired characteristics which are in a format permitting further expansion or testing after selection. Desirable cell characteristics include, but are not limited to the presence/absence of specific cell surface molecules, presence/absence of secretions, presence/absence of intracellular products, cellular morphology, viability, proliferation ability, and/or the presence of desirable cell:cell interaction activity and the like.

The methods of the disclosure are performed on a microfluidic device capable of manipulating microdroplets, in which at least one cell or part(s) of a cell(s) is present. The cell(s) may be natural. The cell(s) may be artificial. The cell(s) may be a microcell(s). At least one liposome may be present.

### Device

The present invention relates to methods capable of being carried out on a microdroplet manipulation device, such as an EWOD or oEWOD device. Any suitable device may be employed that allows for the manipulation of microdroplets according to the methods herein described.

Devices for manipulating droplets or magnetic beads have been previously described in the art; see for example US6,565,727, US2013-0233425 and US2015-0027889. In the case of droplets this is typically achieved by causing the droplets, for example in the presence of an immiscible carrier fluid, to travel through a microfluidic channel defined by two opposed walls of a cartridge or microfluidic tubing. Embedded in the walls of the cartridge or tubing are electrodes covered with a dielectric layer each of which are connected to an A/C biasing circuit capably of being switched on and off rapidly at intervals to modify the electrowetting field characteristics of the layer. This gives rise to localised directional capillary forces that can be used to steer the droplet along a given path.

Electrowetting-on-dielectric (EWOD) is a well-known effect in which an electric field applied between a liquid and a substrate makes the liquid more wetting on the surface than the natural state. The effect of electrowetting can be used to manipulate (e.g., move, divide, or change shape of) fluids by applying a series of spatially varying electrical fields on a substrate to increase the surface wettability following the spatial variations in a sequence. Droplets manipulated in electrowetting-based devices are typically sandwiched between two parallel plates and actuated by digital electrodes.

A variant of this approach, based on optically-mediated electrowetting, has been disclosed in for example US6,958,132, US2015-0298125 and US9,815,056. An improved version of this approach which enables many thousands of microdroplets, generally in the size range less than 10µm, to be manipulated simultaneously is described in WO2018/234445.

In this oEWOD device, the microdroplets are translocated through a microfluidic space defined by containing walls; for example a pair of parallel plates having the microfluidic space therebetween. At least one of the containing walls includes what are referred to as 'virtual' electrowetting electrodes locations which are generated by selectively illuminating an area of a semiconductor layer buried within the wall. By selective illumination of the layer with light from a separate light source, a virtual pathway of virtual electrowetting electrode locations can be generated transiently along which the microdroplets can be caused to move. Further, a suitable oEWOD device is described in WO2020/104769, which may be particularly relevant for the method since it is particularly relevant when the microdroplets contain cells. Other oEWOD devices are described in WO2020/169965.

Another disclosure of oEWOD is the single sided open configuration platform of Park, Sung-Yong, Michael A. Teitell, and Eric PY Chiou, "Single-sided continuous optoelectrowetting (SCOEW) for droplet manipulation with light patterns." Lab on a Chip 10.13 (2010): 1655-1661.

A device suitable for manipulating microdroplets may optionally include one or more of the following features:
a first composite wall comprised of:
   ▪ a first transparent substrate
   ▪ a first transparent conductor layer on the substrate having a thickness in the range 70 to 250nm;
   ▪ a photoactive layer activated by electromagnetic radiation in the wavelength range 400-1000nm on the conductor layer having a thickness in the range 300-1000nm and
   ▪ a first dielectric layer on the conductor layer having a thickness in the range 120 to 160nm;
a second composite wall comprised of:
   ▪ a second substrate;
   ▪ a second conductor layer on the substrate having a thickness in the range 70 to 250nm and
   ▪ optionally a second dielectric layer on the conductor layer having a thickness in the range 120 to 160nm
such that the exposed surfaces of the first and second dielectric layers are disposed at least 10µm, preferably 50-100µm apart to define a microfluidic space adapted to contain microdroplets; and either of the dielectric layers are coated with a biocompatible coating or an anti-fouling coating.

Optionally there may be an interstitial layer of silica of thickness 1-10nm in between the dielectric layers and the anti-fouling/biocompatible coatings;
an A/C source to provide a voltage across the first and second composite walls connecting the first and second conductor layers;
at least one source of second electromagnetic radiation having an energy higher than the bandgap of the photoexcitable layer adapted to impinge on the photoactive layer to induce corresponding virtual electrowetting electrode locations on the surface of the first dielectric layer and
means for manipulating the points of impingement of the electromagnetic radiation on the photoactive layer so as to vary the disposition of the virtual electrowetting electrode locations thereby creating at least one optically-mediated electrowetting pathway along which the microdroplets may be caused to move.

Methods and apparatus for high throughput microdroplet manipulation is described in co-pending application PCT/GB2021/050148 and such apparatus and techniques would be suitable for performing the methods of the present disclosure. The apparatus disclosed therein includes two individually controllable optical assemblies, both of which are capable of generating fixed and switchable arrays of light spots on the surface of the oEWOD chip. Such a configuration is optimised to facilitate high throughput, flexible loading and processing of microdroplets. The first optical assembly may form a plurality of oEWOD traps on a surface of the chip to cause a plurality of microdroplets on the surface of the chip to form an array of microdroplets corresponding to a first array of oEWOD traps; and the second optical assembly may form a second array of oEWOD traps on the surface of the chip. One or more of the oEWOD traps of the second array are aligned with the oEWOD traps of the first array. The method may involve inspecting the contents of the array of microdroplets and making an adjustment to the first optical assembly whilst one or more of the microdroplets are held in place by second array of oEWOD traps.

In some embodiments, there is provided a device for manipulating a microdroplet using optically-mediated electrowetting, the device comprising:
- a first composite wall comprising:
   - a first substrate;
   - a first conductor layer on the substrate;
   - a photoactive layer on the conductor layer; and
   - a first dielectric layer on the photoactive layer having a thickness of less than 20 nm;
- a second composite wall comprising:
   - a second substrate;
   - a second conductor layer on the substrate; and
   - a second dielectric layer on the second conductor layer having a thickness of less than 20 nm.

The first and second dielectric layers may be a continuous layer. The first dielectric layer may be deposited onto the photoactive layer by atomic layer deposition. Additionally or alternatively, the second dielectric layer may be deposited onto the photoactive layer.

It has surprisingly been discovered that by providing the first and/or second dielectric layers with a continuous layer of thickness of less than 20 nm results in the droplets being more stable and therefore, the droplets are stationary on the substrate. In contrast, the inventors have found that increasing the first and/or second dielectric layers to a thickness of above 20 nm can result in more uncontrolled droplet movement on the substrate and therefore, droplets are more likely to exhibit uncontrolled motion deviating from the illuminated regions. As a consequence, uncontrolled droplets can make it more difficult for accurate and efficient oEWOD operations for example, merging or splitting of droplets.

In some embodiments, the first and/or second dielectric layer may be a thickness of between 1 nm to 20 nm, or it may be 2 nm to 20 nm, 3 nm to 20 nm, 4 nm to 20 nm, 5 nm to 20 nm, 6 nm to 20 nm, 7 nm to 20 nm, 8 nm to 20 nm, 9 nm to 20 nm, 10 nm to 20 nm, 12 m to 20 nm, 14 nm to 20 nm, 15 nm to 20 m or 18 nm to 20 nm. It may also be 1 to 15 nm, 1 to 10 nm, 1 to 5 nm, 5 to 10 nm, 5 to 15 nm or 10 to 15 nm.

The first substrate and the first conductor layer and/or the second substrate and the second conductor layer may be transparent.

The device may further comprise an A/C source to provide a voltage across the first and second composite walls connecting the first and second conductor layers; at least one source of electromagnetic radiation having an energy higher than the bandgap of the photoexcitable layer adapted to impinge on the photoactive layer to induce corresponding ephemeral electrowetting locations on the surface of the first dielectric layer; and a microprocessor for manipulating the points of impingement of the electromagnetic radiation on the photoactive layer so as to vary the disposition of the ephemeral electrowetting locations thereby creating at least one electrowetting pathway along which the microdroplet may be caused to move.

The device may further comprise an interstitial layer of silicon oxide. The advantage of the interstitial layer is that it can be used as a binding layer for a anti or non-fouling layer. The interstitial layer is provided between the dielectric layer and the hydrophobic layer. The thickness of the interstitial layer may be between 0.1 nm to 5 nm. The thickness of the interstitial layer can be more than 0.1, 0.25, 0.5, 0.75, 1, 1.5, 2, 2.5, 3, 3.5, 4 or 4.5 nm, or it may be less than 5 nm, 4.5, 4, 3.5, 3, 2.5, 2, 1.5, 1, 0.75, 0.5 or 0.25 nm.

The exposed surfaces of the first and second dielectric layers may be disposed less than 200 µm apart to define a microfluidic space adapted to contain the microdroplet. The microfluidic space may be between 2 and 50 µm in width. In some embodiments, the microfluidic space is more than 2, 4, 6, 8, 10, 12, 14, 16, 18, 20, 22, 24, 26, 28, 30, 32, 34, 36, 38, 40, 42, 44, 46 or 48 µm. In some embodiments, the microfluidic space may be less than 50, 48, 46, 44, 42, 40, 38, 36, 34, 32, 30, 28, 26, 24, 22, 20, 18, 16, 14, 12, 10, 8, 6 or 4 µm.

The exposed surfaces of the first and second dielectric layers may include one or more spacers for holding the first and second walls apart by a predetermined amount to define a microfluidic space adapted to contain the microdroplet. The physical shape of the spacers may be used to aid the splitting, merging and elongation of microdroplets in the device. In some embodiments, the microdroplets may contain one or more cells. The microdroplets may also contain a media, such as a cell media and/or a buffer solution.

The A/C source may be configured to provide a voltage of between 0V and 100V across the first and second composite walls connecting the first and second conductor layers. In some embodiments, the A/C source can be configured to provide a voltage of more than 0, 5, 10, 15, 20, 25, 30, 35, 40, 50, 60, 70, 80 or 90 V or it may be less than 90, 80, 70, 60, 50, 45, 40, 35, 30, 25, 20, 15, 10 or 5V.

The first and second composite walls may further comprise first and second anti-fouling layers on respectively the first and second dielectric layers. The anti-fouling layer on the second dielectric layer may be hydrophobic. The source(s) of electromagnetic radiation may comprise a pixellated array of light reflected from or transmitted through such an array. The electrowetting locations may be crescent-shaped in the direction of travel of the microdroplets. The device may further comprise a photodetector to detect an optical signal in the microdroplet located within or downstream of the device. The optical signal may be a fluorescence signal. The device may further comprise an upstream inlet to generate a medium comprised of an emulsion of aqueous microdroplet in an immiscible carrier fluid.

The device may further comprise an upstream inlet to induce a flow of a medium comprised of an emulsion of aqueous microdroplet in an immiscible carrier fluid through the microfluidic space via an inlet into the microfluidic space.

The first and second composite walls which define the microfluidic space therebetween may form the periphery of a cartridge or chip. The device may further comprise a plurality of first electrowetting pathways running concomitantly to each other. The device may further comprise a plurality of second electrowetting pathways adapted to intersect with the first electrowetting pathways to create at least one microdroplet-coalescing location.

The device may further comprise an upstream inlet for introducing the microdroplet into the microfluidic space, in which the diameter of the microdroplet is more than 20% greater than the width of the microfluidic space.

The second composite wall may further comprise a second photoexcitable layer and the source of electromagnetic radiation may also impinge on the second photoexcitable layer to create a second pattern of ephemeral electrowetting locations which can also be varied.

The source of electromagnetic radiation may be an LED light source, which may provide electromagnetic radiation at a level of 0.005 to 0.1Wcm⁻². In some embodiments, the source of electromagnetic radiation is at a level of 0.005 to 0.1Wcm⁻², or it could be more than 0.005, 0.0075, 0.01, 0.025, 0.05 or 0.075 Wcm⁻². In some embodiments, the source of electromagnetic radiation is at a level may be less than 0.1, 0.075, 0.05, 0.025, 0.01, 0.0075, 0.005 or 0.0025 Wcm⁻².

The first transparent conductor layer on the substrate may be a thickness in the range 70 to 250nm. The photoactive layer may be activated by electromagnetic radiation in the wavelength range 400 to1000nm on the conductor layer, which may have a thickness in the range 300 to 1000nm. In some embodiments, the photoactive layer can be made out of amorphous silicon.

In some embodiments, microdroplets can be passed through a microfluidic space defined by two opposing walls where each of the walls includes a dielectric layer with a sufficiently low voltage applied across the dielectric layers so as to be below the dielectric breakdown voltage of the dielectric layers. The use of the two dielectric layers with a sufficiently low voltage across the dielectric layers not only prevents destructive ionization of conductive droplets but substantially eliminates the adverse effects of dielectric pinhole defects on the droplets which unexpectedly improves performance notwithstanding the reduction in electrowetting forces resulting from the use of two dielectric layers. As a consequence, the optically-mediated electrowetting can be achieved using a low power source of illumination such as, for example, LEDs generating as low as 0.01W/cm² for simultaneously manipulating thousands of droplets. In the case of an embodiment comprising a large-area microfluidic device with area greater than 1cm x 1cm, the device is suitable for manipulating more than 10,000 droplets in parallel, more than 50,000 droplets, more than 100,000 droplets or more than 1,000,000 droplets in the case of a very large-area device.

In some embodiments, a large area device can be utilised for handling many thousands of droplets. The inventors had tried to previously build a larger device using a single dielectric layer for handling droplets in parallel, however the inventors encountered defective areas where droplets could not move. Through experimentation and test, the inventors found pinhole defects to be an important limitation of device performance, especially as devices became larger.

The dielectric layers always have sparse pinhole defects, whereby they become conducting in a small, isolated region. Known optimized processes can give densities of circa 38 pinholes per cm². A pinhole defect can trap a droplet and make it impossible to move. The effect is more profound when using droplets of conducting media such as buffer solutions.

In some embodiments, there is provided a two dielectric layer structure that can be used below the dielectric breakdown. When run below the breakdown voltage, the two sided dielectric layer structure can give the novel effect of largely negating the effect of pinhole defects. With the dielectric disposed over both top and bottom of the droplets, a conducting path could only be formed if a pinhole defect in the first dielectric layer directly lined up with a pinhole defect in the second dielectric layer. The probability of this occurring is very, very small. This pinhole-mitigation feature achieved by the presence of the second dielectric layer is key to permitting the simultaneous manipulation of thousands of droplets in a relatively large area.

In the case of a large area device or a very large area device suitable for manipulating more than 100,000 droplets or even more than 1,000,000 droplets in parallel the number of pinhole defects becomes an important limitation in the device performance, because the probability of a single droplet contacting a pinhole defect becomes exceptionally high. A single droplet trapped on a pinhole defect may block the motion of other droplets in the device and so impair or interrupt the operation of the system. As such the advantage of the present invention in negating the effect of pinhole defects is exceptionally important in the operation of a very large area device containing large numbers of microdroplets.

Referring to Fig 18, there is provided a microfluidic device and in particular, an oEWOD device **100.** The oEWOD device as illustrated in Fig 18 comprises: a first composite wall **102** comprised of a first substrate **104,** which can be made out of glass, a first transparent conductor layer **106** on the substrate **104,** the first transparent conductor layer **106** having a thickness in the range 70 to 250nm; a photoactive layer **108** activated by electromagnetic radiation in the wavelength range 400-850nm on the conductor layer **106,** the photoactive layer **108** having a thickness in the range 300-1500nm and a first dielectric layer **110** on the photoactive layer **108.** The first dielectric layer **110** is formed as a continuous layer that has a thickness of less than 20nm. The lower bound for the thickness of the layer will be dictated, at least in part, by the methodology of providing such a thin layer that must be continuous. However, theoretically it could have a thickness of between 0.1 nm to 20 nm.

The device **100** also comprises a second composite wall **112** comprising: a second substrate **114,** which can be made out of glass and a second transparent conductor layer **116** on the substrate **114.** The second conductor layer **116** may have a thickness in the range 70 to 250nm. A second dielectric layer **118** may be on the transparent second conductor layer **116,** where the second dielectric layer **118** has a thickness of less than 20nm. As with the first dielectric layer, the second dielectric layer must be continuous and the practical lower bound for the thickness is dictated by manufacturing constraints although it could be between 1 nm to 20 nm. The exposed surfaces of the first **110** and second **118** continuous dielectric layers are disposed 20 to 180µm apart to define a microfluidic space **121** adapted to contain microdroplets **122.** The photoactive layer **108** is made out of amorphous silicon. The first and second conductor layers are made out of ITO.

An interstitial binding layer **124** is provided on the first dielectric layer **110** and can also be provided on the second dielectric layer **118.** The thickness of the interstitial layer may be between 0.1 nm to 5 nm. The thickness of the interstitial layer can be more than 0.1, 0.25, 0.5, 0.75, 1, 1.5, 2, 2.5, 3, 3.5, 4 or 4.5 nm, or it may be less than 5 nm, 4.5, 4, 3.5, 3, 2.5, 2, 1.5, 1, 0.75, 0.5 or 0.25 nm. The advantage of the interstitial layer is that it can be used as a binding layer for an anti-fouling or non-fouling layer, which may be hydrophobic.

A hydrophobic layer **126** is provided on the interstitial binding layer **124.** An example of a hydrophobic layer could be a fluorosilane. The interstitial binding layers **124** are optional and the channel walls **120** can be made of SU8, or they may be part of the glass structure. The interstitial layer **124** is provided between the dielectric layer **110, 118** and the hydrophobic layer **126.**

An incident light **130,** as illustrated in Fig 18, can be used to provide a light sprite pattern **131** in which the incident light **130** provides light onto a portion of the photoactive **110** to hold the microdroplet **122** into a stationary position within the microfluidic space **121.** An oil carrier phase **134** can be provided to the microdroplets **122,** through a hole **136** in the device, to replenish key nutrients and components to keep the contents within the microdroplet **122,** such as one or more cells, alive and healthy. In some cases, the oil phase **134** can provide key nutrients, media and contents for cell growth, viability and/or productivity.

The first and second substrates **104, 114** are made of a material which is mechanically strong. For example, the first and second substrates can be formed from glass, metal or an engineering plastic. In some embodiments, the substrates may have a degree of flexibility. In some embodiments, the first substrate is silicon, fused silica or glass. In some embodiments, the second substrate is fused silica and glass.

The first and second conductor layers **106, 116** are located on one surface of the first and second substrates **104, 114** and typically have a thickness in the range 70 to 250nm, preferably 70 to 150nm. At least one of these layers is made of a transparent conductive material such as Indium Tin Oxide (ITO), a very thin film of conductive metal such as silver or a conducting polymer such as PEDOT or the like. These layers may be formed as a continuous sheet or a series of discrete structures such as wires. Alternatively, the conductor layer may be a mesh of conductive material with the electromagnetic radiation being directed between the interstices of the mesh.

The photoactive layer **108** is formed from a semiconductor material which can generate localised areas of charge in response to stimulation by the source of electromagnetic radiation. Examples include hydrogenated amorphous silicon layers having a thickness in the range 300 to 1500nm. In some embodiments, the photoactive layer is activated by the use of visible light. The dielectric properties of this layer preferably include a high dielectric strength of >10^7 V/m and a dielectric constant of >3. In some embodiments, the dielectric layer is selected from alumina, silica, hafnia or a thin non-conducting polymer film.

Alternatively, at least the first dielectric layer, preferably both, may be coated with an anti-fouling layer to assist in the establishing the desired microdroplet/carrier fluid/surface contact angle at the various virtual electrowetting electrode locations. The anti-fouling layer is intended additionally to prevent the contents of the microdroplets adhering to the surface and being diminished as the microdroplet is moved through the chip. For optimum performance, the anti-fouling layer should assist in establishing a microdroplet/carrier fluid/surface contact angle that should be in the range 50°-180° when measured as an air-liquid-surface three-point interface at 25°C. In some embodiments, these layer(s) have a thickness of less than 10nm and are typically formed as a monomolecular layer. Alternatively, these layers may be comprised of a polymer of an acrylate ester such as methyl methacrylate or a derivative thereof substituted with hydrophilic groups; e.g. alkoxysilyl. Either or both of the anti-fouling layers are hydrophobic to ensure optimum performance. In some embodiments an interstitial layer of silica of thickness less than 20nm may be interposed between the anti-fouling coating and the dielectric layer in order to provide a chemically compatible bridge.

The first and second dielectric layers, and therefore the first and second walls, define a microfluidic space which is at least 10µm, and preferably in the range of 20 to180µm, in width and in which the microdroplets are contained. Preferably, before they are contained, the microdroplets themselves have an intrinsic diameter which is 10% greater or 20% greater, than the width of the microdroplet space. Thus, on entering the chip the microdroplets are caused to undergo compression leading to deformation of the spherical microdroplet that leads to enhanced electrowetting performance through e.g. a better microdroplet merging capability. In some instances, the first and second dielectric layers can be coated with a hydrophobic coating such a fluorosilane.

In some embodiments, the microfluidic space includes one or more spacers for holding the first and second walls apart by a predetermined amount. Options for spacers include beads or pillars, ridges created from an intermediate resist layer which has been produced by photo-patterning. Alternatively, deposited material such as silicon oxide or silicon nitride may be used to create the spacers. Alternatively layers of film, including flexible plastic films with or without an adhesive coating, can be used to form a spacer layer. Various spacer geometries can be used to form narrow channels, tapered channels or partially enclosed channels which are defined by lines of pillars. By careful design, it is possible to use these spacers to aid in the deformation of the microdroplets, subsequently perform microdroplet splitting and effect operations on the deformed microdroplets. Similarly these spacers can be used to physically separate zones of the chip to prevent cross-contamination between droplet populations, and to promote the flow of droplets in the correct direction when loading the chip under hydraulic pressure.

The first and second walls are biased using a source of A/C power attached to the conductor layers to provide a voltage potential difference therebetween; suitably in the range 0 to 50 volts. These oEWOD structures are typically employed in association with a source of electromagnetic radiation having a wavelength in the range 400-850nm, for example 550, 620 and 660 nm and an energy that exceeds the bandgap of the photoactive layer. Suitably, the photoactive layer will be activated at the virtual electrowetting electrode locations where the incident intensity of the radiation employed is in the range 0.005 to 0.1 Wcm⁻². The source of electromagnetic radiation is at a level of 0.005 to 0.1Wcm⁻², or it could be more than 0.005, 0.0075, 0.01, 0.025, 0.05 or 0.075 Wcm⁻². In some embodiments, the source of electromagnetic radiation is at a level may be less than 0.1, 0.075, 0.05, 0.025, 0.01, 0.0075, 0.005 or 0.0025 Wcm⁻².

Where the sources of electromagnetic radiation are pixelated they are suitably supplied either directly or indirectly using a reflective screen such as a digital micromirror device (DMD) illuminated by light from LEDs or other lamps. This enables highly complex patterns of virtual electrowetting electrode locations to be rapidly created and destroyed on the first dielectric layer thereby enabling the microdroplets to be precisely steered along essentially any virtual pathway using closely-controlled electrowetting forces. Such electrowetting pathways can be viewed as being constructed from a continuum of virtual electrowetting electrode locations upon the first dielectric layer.

The first and the second dielectric layers may be composed of a single dielectric material or it may be a composite of two or more dielectric materials. The dielectric layers may be made from, but is not limited to, Al₂O₃ and SiO₂.

A structure may be provided between the first and second dielectric layers. The structure between the first and second dielectric layers can be made of, but is not limited to, epoxy, polymer, silicon or glass, or mixtures or composites thereof, with straight, angled, curved or micro-structured walls/faces. The structure between the first and second dielectric layers may be connected to the top and bottom composite walls to create a sealed microfluidic device and define the channels and regions within the device. The structure may occupy the gap between the two composite walls. Alternatively or additionally, the conductor and dielectrics may be deposited on a shaped substrate which already has walls.

### Microdroplets

The methods of the invention require the manipulation of at least one microdroplet within the device which generally refers to a droplet of liquid, such as an aqueous liquid, containing for example a cell/cells, a reporter entity or a combination of the two. Microdroplets provide compartments in which species or reactions can be isolated. They allow the possibility of working with single cells and are suitable for high-throughput experimentation. Microdroplets permit the encapsulation of the cell and/or reporter entities, keeping the same separate until merging becomes desirable.

A microdroplet can be of any shape or size but preferably, the droplet is of spherical or cylindrical shape. The size of a microdroplet may be between 20 to 600 µm but it may be more than 20, 30, 40, 50, 60, 80, 100, 120, 140, 150, 160, 180, 200, 220, 240, 250, 260, 280, 300, 320, 340, 360, 380, 400, 420, 440, 460, 480, 500, 520, 540, 550, 560 or 580 µm. In some embodiments, the size of the microdroplet may be less than 600, 580, 560, 550, 540, 520, 500, 480, 460, 440, 420, 400, 380, 360, 340, 320, 300, 280, 260, 250, 240, 220, 200, 180, 160, 150, 140, 120, 100, 80, 60, 50, 40 or 30 µm. A plurality of microdroplets can be merged together to form a larger droplet. Alternatively, a large droplet can be divided to form smaller sized microdroplets (child microdroplets) as appropriate.

If the droplet is too small relative to the height of the microfluidic device then the droplet is not in contact with both sides of the walls within the microfluidic chamber and hence, the droplet cannot be moved by oEWOD. In contrast, large droplets with respect to the device geometry can be hard and/or slow to move within the microfluidic device and often disrupt other operations simply by getting in the way by obstructing other correctly-sized droplets or by merging with correctly-size droplets.

In general, microdroplets usually consist of an aqueous phase which contains the content of the microdroplet (such as a cell or cells, nutrients, reporter entities, assay reagents, hydrogel bead polymers). In some cases microdroplets consist of additional interior phases of immiscible fluids surrounding further aqueous material, forming multiple emulsions such as double emulsions and droplet-in-droplet emulsions.

In a microfluidic device, the microdroplets are separated from each other by an immiscible carrier phase or fluid. Suitable carrier phases may include oils, such as silicone oils, minerals oil or fluorocarbon oils. Exemplary fluorocarbon oil includes HFE7500, HFE7700 or FC-40. The carrier phase may suitably further contain surfactants and other additives to maintain microdroplet stability. The surfactant may localise at the microdroplet/carrier interphase and therefore stabilise the microdroplet by reducing surface tension. Where the weight ratio of aqueous microdroplets to carrier is low there is a tendency for the microdroplets to shrink over time; a phenomenon which can lead to a loss of reactivity within them. One way of counteracting this effect is to use a carrier phase which has been hydrated. Since generally the carriers described above do not have a high capacity for dissolving water, hydration is suitably achieved by creating micelles or secondary microdroplets of water or aqueous buffer within the carrier phase. This buffer may have a composition which is the same as or different to that of the microdroplets themselves. In some embodiments these micelles or secondary microdroplets may contain up to five times the salt content of the microdroplets themselves and optionally contain glycerol. Typically these micelles and secondary microdroplets are an order of magnitude smaller. In some situations the carrier phase may contain lipophilic compounds which can diffuse in and out of the dispersed phase droplets.

The microdroplets used in the methods of the disclosure comprise various components, dependent upon which panel they are present in. The microdroplets are aqueous in nature, and may contain additional components, for example components to provide support structures such as gels or coated microspheres. They may further comprise cell culture medium, with a formulation that may include nutrients, a source of energy such as carbohydrates, buffering agents and the like.

The methods of the disclosure comprise the use of a microdroplet which contains at least a single cell. Said cell is the cell which is to be assayed or tested for selection, optionally based on the characteristics of said cell. Given that it is the characteristics of the cell that are to be selected against, the first microdroplet generally contains a single cell. The methods of the disclosure may, therefore, involve the step of selecting only microdroplets for the panel that include a single cell, and disposing of empty or multi-occupancy cells. Subsequent to this step, the cell may be capable of division, such that the microdroplet may contain at least one cell.

The microdroplets containing at least one cell form a panel of microdroplets.

A panel of microdroplets is formed by a plurality of microdroplets. The panel of microdroplets is a collection, group or array of microdroplets, each of which have similar contents. For example, in the panel of microdroplets comprising a cell, each of said microdroplets may comprise at least one cell, optionally of the same type (such as a B cell). The panel of microdroplets alternatively may comprise cell culture medium, reporter entities, selected cells, expanded cells, nucleic acid and the like. A panel of microdroplets may comprise, for example, 50 - 700,000 microdroplets.

In the methods of the present disclosure, a panel of microdroplets comprising at least one reporter entity is provided. Such microdroplets may contain at least one reporter entity as defined further herein.

The methods of the present disclosure involve the selection of a subset of a panel of microdroplets. This selected subset may then form a new panel of microdroplets for further use in the methods of the disclosure.

The microdroplets are manipulated in the device using real or virtual electrowetting electrodes. The microdroplets may be manipulated into arrays for ease of arranging merging, splitting and detection events. The merging and splitting events may be performed using real or virtual electrowetting electrodes. Any suitable layout may be used to manipulate the microdroplets to achieve the desired methods. The microdroplets may also be manipulated using alternative methods, such as acoustically.

The microdroplets are generally prepared by encapsulating the aqueous preparation with required components (such as the cell, reporter entities). The microdroplets are formed via emulsification techniques. In one example the emulsification is a vortex emulsification. In another example the emulsification is a step emulsification. The microdroplets may be created on the microfluidic device, or may be generated adjacent thereto.

### Cell

The methods of the present disclosure are for the selection of cells with particular or desirable characteristics. For the avoidance of doubt, said cells are biological cells. The cell(s) may be natural. The cell(s) may be artificial. The cell(s) may be microcells. The methods of the invention may be cell free and use part(s) of a cell(s), for example nuclei and/or mitochondria. The methods of the invention may use liposomes. Said cells may be taken from any appropriate source, for example a cell sample from a human or animal, plant or microorganism.

The cell may be a cell from a human or animal, optionally a mammal. The cell may be a plant cell, insect cell, fungal cell, bacterial cell or ameobal cell. The cell may be a cell-fusion such as a hybridoma.

The cells may be taken from a cell culture, for example a culture of stem cells, pluripotent cells, genetically engineered cells and the like.

If the cell is derived from a sample/biological sample this may be any human, animal, environmental (natural, contrived or modified), or food sample containing at least one cell type. The sample/biological sample may be selected from: stool, peripheral blood, sera, plasma, ascites, urine, cerebrospinal fluid (CSF), sputum, saliva, bone marrow, synovial fluid, aqueous humour, amniotic fluid, cerumen, breast milk, broncheoalveolar lavage fluid, semen, prostatic fluid, cowper's fluid or pre-ejaculatory fluid, female ejaculate, sweat, faecal matter, hair, tears, cyst fluid, pleural and peritoneal fluid, pericardial fluid, lymph, chyme, chyle, bile, interstitial fluid, menses, pus, sebum, vomit, vaginal secretions, mammary secretions, mucosal secretion, stool water, pancreatic juice, lavage fluids from sinus cavities, bronchopulmonary aspirates, blastocyl cavity fluid, and umbilical cord blood. Alternatively, the sample may come from a tissue sample.

The cell may be isolated from a patient or individual. The methods described here may be used to screen such cells and return them to the patient (autologous cell transfer). The cells may be isolated from one individual and selected to be administered to a patient (allogenic cell transfer).

For some embodiments, the panel of microdroplets containing at least one cell contain cells of the same type, for example lymphocytes such as T cells. Therefore, the cells may be pre-selected prior to their inclusion into microdroplets. However, some contamination may occur with any biological cells wherein cells of a different type may also be included within the microdroplets, for example, B cells when T cells are the desired type. The methods of this disclosure will quickly identify such microdroplets, such that they are not selected for the subset of microdroplets.

For some embodiments, there may be a diverse population of cell types included in the panel of microdroplets, such as if an environmental sample is being screened with unknown bacterial cells present.

The cell may be a human or mammalian cell. The cell may be any suitable type from any tissue type, such as from organ or tissue of the body.

The cell may be an immune system cell. Such cells include monocytes, macrophages, osteoclasts, neutrophils (polymorphonuclear leukocytes) dendritic cells, microglial cells, mast cells, T cells (including helper T cells, regulatory T cells, cytotoxic T cells and natural killer T cells), B cells, natural killer cells and hematopoietic stem cells.

The cell may be a pluripotent or stem cell, isolated or prepared via culturing techniques. The pluripotent stem cells may be reprogrammed mature cell types.

The cell may be genetically engineered prior to encapsulation into the microdroplet. The cell may be genetically engineered after encapsulation in the microdroplet.

Genetic engineering of the cell may be by any suitable method, including transduction (viral gene transfer), gene editing (using a nuclease such as zinc finger nucleases, TALEN, CRISPR/Cas9 base and prime editing) non-viral gene delivery (such as nanoparticle delivery), gene knock down, gene knock in and gene manipulation using RNA, for example, such as gene silencing or activation, or optogenetics. The genetic engineering generally involves the introduction of a genetic element into the cell, by any suitable means.

The genetic engineering may simply be the introduction of entities that cause mutations within the genome of the cell to see whether random mutations result in desirable cell characteristics. The methods of the present disclosure permit the screening of large number of cells with random or intentional mutations, such that cells with desirable characteristics following mutation may be selected. Such permits optimisation of cell selection, particularly for cell-based manufacturing. Such may be described as "directed evolution" of the cells.

The genetic element optionally comprises a nucleic acid that is operably linked to a promotor, wherein the nucleic acid sequence encodes a desirable product, such as a CAR.

The genetic element may be a viral vector containing a sequence of interest which it is desired to express or include in the cell. The nucleic acid may be a non-viral "naked" vector such as a minicircle for expression in the cell. The nucleic acid may include gene editing components to edit the gene in a cell.

The genetic element may include any desirable sequence to permit any desirable genetic engineering to be made to the cell, for example to express a new cell surface molecule.

In one embodiment, the cells may be genetically engineered whilst encapsulated into a microdroplet. In this embodiment, a panel of microdroplets comprising at least one cell and a panel of microdroplets comprising at least one genetic element is provided. The method of the disclosure may therefor comprise a further step of merging microdroplets of the panel comprising at least one cell with microdroplets of the panel comprising at least one genetic element to form a merged panel of microdroplets. Such microdroplets contain at least one genetically engineered cell that may be subjected to the methods of selection described herein. Assay can then determine the success of the genetic engineering and non-modified cells may be discarded.

The genetic engineering may involve the provision of a gene sequence under an inducible promoter. This permits the engineered cells to be stimulated to produce the product encoded by the gene sequence.

When preparing a panel of microdroplets containing a cell, some microdroplets may be empty, since the encapsulation step may result in the encapsulation of medium only, or the cell may die between the encapsulation and method steps. Alternatively, the splitting step may result in the lack of a cell in a microdroplet. In this process the encapsulation of the cells will results in a population of droplets with occupancy described by Poissonian statistics.

The methods of the present disclosure may be used with actively-controlled droplet production methods to improve the efficiency of single cell encapsulation. Optionally the encapsulation process provides a panel of microdroplets which contain a single cell. Single cell encapsulation permits clonal expansion during the process.

However, some encapsulation processes that can be utilised may not be as efficient; the number of cells encapsulated per droplet in these systems is dictated by Poisson statistics, reducing the proportion of droplets that contain the desired number of cells and thus the effective rate at which single cells can be encapsulated. Thus, it is possible that the encapsulation process selected by the user of the device may result in a minority of cells that contain one or more cells. However, those skilled in the art are tolerant of such a situation, and these empty microdroplets may be subjected to the same splitting and merging operations, and discounted for the purposes of selection. Thus, the selection methods described herein are flexible and can be utilised with various methods of cell encapsulation.

Thus, wherein the term "panel of microdroplets containing at least one cell" is used, it will be appreciated by those skilled in the art that a proportion may lack one or more cells. The methods of the present disclosure may quickly identify such empty microdroplets where a cell is required and discard them from further consideration. Thus in the panel of microdroplets containing at least one cell, substantially all of the microdroplets may contain at least one cell, but some or a proportion may be empty. The panel of microdroplets may also deliberately contain empty microdroplets as a control or location marker.

The panel of microdroplets may also contain control microdroplets, such as microdroplets containing cells with known characteristics or known products/molecules such as antibodies, such that they may provide a positive control for the reporter entities. Negative controls may also be included; this could be a cell or product/molecule that is not expected to generate a positive result with the reporter entities, or this could be an empty droplet or a droplet of media.

Similarly, wherein the term "a panel of microdroplets containing one or more reporter entities" is used it will be appreciated by those skilled in the art that a proportion may lack one or more reporter entities. The methods of this present disclosure may identify such empty microdroplets and remove any which do not contain the required reporter entity; however some droplets not having the required reporter entity may still be present in the assay. In some cases droplets without reporter entities may be introduced to a subset of the panel to act as a negative control. Substantially all of the panel may comprise a reporter entity.

### Cell Characteristics

The cells to be selected may be chosen on the basis of one or more characteristics.

Such characteristics may include the nature of and/or the presence or absence of any one or more of:
a cell surface molecule, activation profile, ability to proliferate, secretion ability, affinity of secreted product, functional behaviour of secreted products, ability to make intracellular products, viability, morphology and/or cell:cell interaction ability (such as cell killing or cell activation or cell aggregation).

The cells may be selected on the basis of any desirable combination of characteristics, which may be tested for substantially simultaneously (multiplex) or sequentially. Therefore, more than one reporter entity may be required, or more than one panel of reporter microdroplets containing at least one reporter entity may be required. Successive rounds of selection may permit selection of a subset of microdroplets containing at least one cell.

In one embodiment, the characteristics that are determined are the presence or absence of one or more cell surface molecules or markers. All cells express characteristic molecules markers (for example: proteins, lipids, glycosylated markers,) that can be used to help distinguish cell types. Specific combinations of cell surface molecules or markers can be used to identify particular cells.

In one embodiment, the cell surface marker is a CD molecule. CD stands for cluster of differentiation (also known as cluster of designation or classification determinant and often abbreviated as CD). Phenotyping of cells is possible using CD molecules. Such markers are often, but not exclusively, associated with certain immune functions. Exemplarily, various T cells may be identified by the presence of CD3, CD4 or CD8; B cells by CD19 or CD20 and NK cells by CD56.

In one embodiment, the cell surface molecule or marker is a cell surface receptor, a cell surface transporter, a cell adhesion protein, a cell surface signalling molecule, and cell surface molecules responsible for cell:cell interactions.

In one embodiment, the cell surface marker is an artificial, synthetic or chimeric cell surface marker that the cell has been genetically engineered to produce.

Optionally, the artificial cell surface marker is a chimeric antigen receptor (CAR). CARs may be composed of four regions: an antigen recognition domain, an extracellular hinge region, a transmembrane domain, and an intracellular signalling domain. The antigen recognition domain is exposed to the outside of the cell, interacting with potential target molecules and is responsible for targeting the modified cell to any cell expressing a matching molecule. The antigen recognition domain is typically derived from the variable regions of a monoclonal antibody linked together as a single-chain variable fragment (scFv), but can also be derived from engineered single domain. Non-antibody approaches have also been used to develop CARs, such as using ligand/receptor pairs that usually bind to each other.

The cell surface markers (natural or modified) may be used to select cells during the selection to define the first subset of cells, or a second or further subset of cells.

In one embodiment, cell selection may be based upon a cell surface marker or molecule. Thus, the reporter entity may be one that interacts with a cell surface marker or molecule.

In one embodiment, the characteristics that are determined are the presence or absence of one or more secretions from the cell. Many cells are capable of secretion, for example mammalian cells are continuously excreting proteins into the extracellular matrix. Different specialised cells may have specialised secretions, for example immune cells may express secretory proteins such as cytokines, immunoglobulins, ligands and receptors. The cell may secrete a protein, including a glycoprotein or lipoprotein, a chemical, a nucleic acid, a polymer or a molecule. Secretion is the step of moving material from the interior of the cell to its exterior, and this may use various pathways dependent upon the type of cell. For example, extracellular vesicles may be involved in any of the embodiments of the invention. Bacterial cells may rely upon secretion abilities to adapt and survive.

The secretion may be an inherent secretion of the cell, for example an immunoglobulin such as an antibody from a B cell, or the secretion may be due to genetic engineering of the cell introducing a gene or coding sequence for a secretion. In one embodiment, the cell is a B cell or hybridoma and the secretion is a monoclonal antibody.

The secretion may or may not have the desired characteristics, for example binding ability. Such may be interrogated by one or more reporter entities.

In one embodiment, cell selection is may be based upon a secretion from the cell. Thus, the reporter entity may be one that interacts with a cell secretion. The cell secretion may be separated from the cell by virtue of splitting a microdroplet into a reference microdroplet containing at least one cell and a test microdroplet containing the medium in which the secretion is present.

In one embodiment, the characteristics that are determined are the presence or absence of one or more intracellular products produced by cell. Most, if not all, cells are capable of being used to produce proteins including glycoproteins and lipoproteins, but some cells, in particular bacteria, are capable of making chemicals, nucleic acids and other polymers. Genetically engineered cells are altered to make a desired product, which may not be secreted but be retained within the cell, such that the cell must be lysed to release it.

The intracellular product may or may not have the desired characteristics, for example catalytic ability. Such may be interrogated by one or more reporter entities.

The product may be an inherent product of the cell, or the product may be due to genetic engineering of the cell introducing a gene or coding sequence for said product.

In one embodiment, cell selection is may be based upon the detection of the intracellular product. Thus, the reporter entity may be one that interacts with said product. The product may be detected in *situ* by a reporter entity that may enter the cell, or the product may be separated from the cell by virtue of cell lysis within the test microdroplet.

The intracellular product may be any suitable biological or chemical molecule. The intracellular product may be a protein, such as a hormone, enzyme, cell signalling molecule, signal transduction molecule, immunoglobulins and the like. The intracellular product may be a nucleic acid, such as RNA, DNA or a hybrid thereof. The intracellular product may be a chemical, such as a pesticide, a toxin, an antibiotic, a fuel, a pharmaceutical drug, a vaccine, an antiviral and the like.

In one embodiment, the cells are selected on the basis of proliferation ability. Such a selection may be determined by detecting the presence of a cell division event (increase in number of cells in the microdroplet) or on the basis of proliferation markers. Proliferation markers may be detected using reporter entities specific therefor.

In one embodiment, the cells are selected on the basis of morphology. Such a selection may be determined by visual inspection of the cells, for example using an appropriate optical detection means. The morphology of the cell includes any one or more of the size, shape, presence/absence of cell adhesion properties, presence/absence of protrusions in the cell membranes such as blebs, presence/absence of intracellular entities such as vacuoles.

In one embodiment, the cells are selected on the basis of the results of the interaction with a reporter entity, wherein the reporter entity is a cell. The interaction may be between the cell and the reporter entity, or a secretion and the reporter entity. The reporter entity cell may be any cell type, such as a tumour cell, a cell line, a tumouroid, a bacterial cell, an immune system presenting cell (such as an antigen presenting cell). In response to the reporter entity cell, the cell to be selected may be activated, may be induced to secrete molecules, and/or may promote cell activation or death of the reporter entity cell, or the secretion may bind thereto.

The selection of cells based on the results of their direct or indirect (via a secretion) interaction with a reporter entity cell may permit characteristics suitable for therapeutic indications to be determined.

The cells may be selected on the basis of any number of desirable characteristics, and/or the absence of undesirable characteristics. These may be based on any combination of the characteristics described here.

### Reporter

The methods described herein relate to the use of at least one panel of microdroplets comprising at least one reporter entity. Any suitable number of panels may be employed, such as 2, 3, 4, 5, 6, 7, 8, 9, 10 or more panels. Each panel may include one or more reporter entities, each of which is different.

Any panel of microdroplets comprising at least one reporter entity may contain 1, 2, 3, 4, 5, 6, 7, 8, 9, 10 or more reporter entities.

A reporter entity is any suitable entity that can interact with the cell, cell product or cell secretion in order to provide a detectable change. The reporter entity may be any one or more of the following entities or a derivative thereof:
i. an antibody;
ii. an antigen;
iii. a receptor;
iv. a ligand;
v. a substrate;
vi. an enzyme;
vii. a ligand;
viii. a nucleic acid;
ix. a cell;
x. a part of a cell;
xi. an extracellular vesicle;
xii. a liposome;
xiii. a polymer;
xiv. a chemical;
xv. a drug;
xvi. a FRET reporter;
xviii. a chemiluminescent material;
xviii. a sample of tissue;
xix. a virus or bacteriophage;
xx. a cytokine; and/or
xxi. a protein.

The reporter entity is capable of interacting with the cell or a part or derivative thereof (such as a product or secretion) and it is this interaction causes a detectable change in the reporter entity. Such a change may be binding, annealing, aggregation, separation, amplification, conformational, activation and/or destruction. The reporter entity may be suitable for direct monitoring for a detectable change, such as in the case of a reporter entity cell. The reporter entity may be associated with/conjugated to structures such as beads or labels, such as a visible, chromogenic, luminescent, fluorescent or phosphorescent label, protein complementation such as split fluorescence, split luminescence or fluorogenic ligands.

. Alternatively or additionally, the change in the reporter entity may be detected by a second entity, which may be labelled (such as a visible, luminescent, fluorescent or phosphorescent label). The second entity may detect, for example, the binding of the reporter entity to the cell, and be a secondary antibody or derivative thereof. Alternatively or additionally, the reporter entity may be conjugated or linked to an enzyme, and the substrate for that enzyme may be added as a second entity, which elicits a visual, chromogenic or fluorescent signal for detection. Those skilled in the art will be aware of the numerous assay means to determine the presence of a particular analyte.

Commonly, the reporter entity will be an antibody or fragment or derivative thereof (such as antigen-binding fragments (Fab), single chain variable fragments (scFv), miniaturised antibodies or affibodies).

In one embodiment, the test panel of microdroplets are contacted with a panel of microdroplets containing a plurality of reporter entities. Thus, the cells may be subjected to a "multi-element reporter assay" or "multi-reporter cell-surface marker assay". Such are assays in which a multitude of characteristics are interrogated by a system of reporter entities, each of which is capable of generating an individual detectable change.

In an embodiment, at least one of the reporter panels comprise at least one reporter entity cell. The cell may be any suitable cell type, including a tumour cell, a cancer cell, a cell line, a tumouroid, a presenting cell, an accessory cell, or an "off target" cell that interaction with would be undesirable. A change in the reporter entity cell is detected. Such a change may be cell death or destruction, cell activation, or cell aggregation. These changes may be observed visually (through appropriate magnification) or through a second entity which detects the presence of an appropriate analyte in the microdroplet, such as internal cell components, cell signalling molecules such as cytokines or presence of upregulated cell surface molecules. Alternatively or additionally, the reporter entity cell may be genetically engineered to include at least one reporter gene which is linked to an event, such as receptor activation. Once this event occurs, an intracellular signalling cascade activates the reporter gene which generates a measurable signal (for example, a luciferase gene, which when provided with the necessary substrate generates light).

In an embodiment, at least one of the reporter panels comprise at least one or more of the following:
i. antigen or ligand-conjugated beads and fluorescent-dye conjugated secondary antibodies;
ii. secondary-antibody-conjugated beads and fluorescent-dye conjugated antigens or ligands; or
iii. antigen or ligand conjugated to a carrier surface and fluorescent-dye conjugated secondary antibodies beads

Such reporter entities are useful in the detection of molecules that are capable of binding, such as antibodies, receptors, enzymes and the like. Those skilled in the art of assay design will be aware of all possible combinations of reporter entities and how characteristics can be detected and reported.

For CAR-T cells, the following entities are suitable characteristics: CD4, CD8, CD19, scFv domain markers (for specificity and/or affinity screening), B-cell markers (or markers for other unwanted contaminant cells) and/or differentiation markers (to choose relevant sub-panels).

### Detection

The change in the reporter entity is capable of detection. As described above, the detection may be a simple observation of an event that is visible, albeit with magnification (microscopy). Thus, the detection event may be a visual detection of the change.

The detection for several embodiments is optical, for example as the label employed may be visual, chromogenic, luminescent, fluorescent or phosphorescent. Suitable detection techniques for all may be employed.

A detection system may be used to screen for signals of each independent reporter entity for each microdroplet. In one example, a panel of merged microdroplets may be imaged and any changes thereto may be detected and quantified. Results of such an assay can be used to determine cell characteristics (phenotype) allowing individual cells to be screened for by the markers they express, the activity they possess, or the products they may or secrete.

A detection system may enable extensive high quality imaging using a high sensitivity camera, for example. In some embodiments, the detection system may support multi-channel fluorescence detection, be capable of dark field imaging, bright field imaging and the detection of cell morphology. High quality imaging may be supported by multiple objective lenses, including high N.A. (60x) lenses. Distinct reporter entity signals may be detected and intensity may be quantified spatially for each microdroplet facilitating the high throughput screening of cells.

### Selection

The methods disclosed herein permit the selection of a subset of cells dependent upon their characteristics. The cells in the microdroplet panel are chosen or selected on the basis of certain characteristics, such as cell surface markers/molecules, internal cell products or secretions from the cell. The characteristics may involve the specificity/activity of the secretion or intracellular product. The chosen or selected cells form a subset of the panel of microdroplets. The remaining microdroplets containing at least one cell may be discarded or discounted. The subset of microdroplets so selected may be subjected to further steps in which the characteristics of the cell are further interrogated or assayed, such that a further subset is selected. Once the final step of the methods described herein is conducted, the final subset of microdroplets containing at least one cell contains the selected cells.

The selected cell or cells may then be dispensed from the device. Once selected and dispensed from the device, the cells may be subjected to further assays, or may be cultured to promote cell maintenance and/or expansion. After dispensing, the cells may optionally be prepared for infusion or re-infusion into a patient. After dispensing the cells may be expanded for use in producing a therapeutic entity, such as an immunoglobulin or pharmaceutical drug.

In one embodiment, the selected cells are present in a reference panel of microdroplets containing at least one cell. The reference panel comprises microdroplets that correlate to a microdroplet in the test panel, since they both originate from the same progenitor microdroplet. In one embodiment, the splitting of the progenitor microdroplet generates a reference panel microdroplet containing at least one cell and a test panel microdroplet containing at least medium. The medium may contain secretions from the cell, which are a characteristic of said cell. In an alternative embodiment, the progenitor microdroplet produces a reference panel microdroplet containing at least one cell and a test panel microdroplet containing at one cell. Said reference panel of microdroplets therefore contains cells which are clones or clonal copies of the panel of microdroplets containing at least one cell which are subjected to assays to determine the cell characteristics. Selection steps may involve the lysing of cells in the test panel, which is not desirable when selecting cells for further expansion.

Thus, according to any embodiment, the results obtained from the assays on the test panel of microdroplets can be directly correlated to cells in the reference panel; such that it is cells from the reference panel that are selected. The advantage of such a step is that the reference panel cells may have not been exposed to some of the reporter entities, which may be deleterious for the cell, or undesirable in a preparation of cells to be used for therapeutic purposes, such as exposure to tumour cells.

The cells may be selected on the basis of the presence of a characteristic, the absence of a characteristic, or a mixture of both. The cells, extracts or secretions therefrom may therefore be tested or assayed for a plurality of characteristics using the methods described herein. In one embodiment, the cells are tested simultaneously for a plurality of characteristics in a single step. Such multiplexed testing is achievable if the change in the reporter entities is detected using different means - such as for example, fluorescent, luminescent and phosphorescent labels or events. Detection means may also comprise protein complementation events such as split fluorescence, split luminescence or fluorogenic ligands.

The selected cells may further be dispensed from the device, such that they may be cultured and permitted to undergo cell division prior to further use. In one embodiment, the cells are for infusion into a patient. In some embodiments, after dispensing the selected panel of microdroplets, the cells contained therein are treated to expand the population and/or to culture clones(s).

After dispensing from the device, the cells and/or non-cellular entities such as secretions, metabolites contained in the subset of microdroplets may be subjected to further analysis, including, but not limited to any one or more of the following:
I. mass spectrometry;
II. surface plasmon resonance;
III. high - performance liquid chromatography; and/or
IV. genetic analysis, including nucleic acid sequencing and PCR amplification.

### Medium

The cells are encapsulated in microdroplets for the methods of the disclosure. The cells according to any embodiment are generally supplied in a medium, usually an aqueous medium. Medium is simply the substance in which the cells are cultured. This medium may be a minimal medium, which contains enough material to support the cells, and the contents of such minimal medium will depend upon the cell type being encapsulated. The ingredients required will vary dependent upon the cell type being studied, and those skilled in the art will appreciate how to identify the correct medium for the task.

In general, cell culture medium may contain a carbon source (for example a sugar such as glucose), water, one or more salts, a pH buffer, a source of amino acids and nitrogen. The medium may be supplemented with molecules or agents that help or promote cell division or cell survival, if required. Such include appropriate nutrients, growth factors, signalling molecules (such as cytokines), vitamins, salts, serum proteins, cofactors, carbohydrates, gases, trace elements and antibiotics.

An exemplary medium is Dulbecco's Modified Eagle's Medium (DMEM), which is a basal medium and contains no proteins or growth promoting agents. Therefore, it requires supplementation to be a "complete" medium. It is most commonly supplemented with 5-10% Fetal Bovine Serum (FBS). DMEM has been found to be widely applicable in mammalian cell culture and can also be used to culture hybridomas. An alternative medium is Eagle's Minimum Essential Medium (EMEM). EMEM contains balanced salt solution, nonessential amino acids, and sodium pyruvate. Since EMEM is a non-complex medium, it is generally fortified with additional supplements or higher levels of serum making it suitable for a wide range of mammalian cells. RPMI is a general purpose media with a broad range of applications for mammalian cells, especially hematopoietic cells. RPMI is a modification of McCoy's 5A and was developed for the long-term culture of peripheral blood lymphocytes and supports the growth of a wide variety of cells in suspension. If supplemented with serum or serum replacement, this medium has a wide range of applications for mammalian cells, including the culture of fresh human lymphocytes, fusion protocols and growth of hybrid cells like mouse hybridoma cells for antibody preparations. It is also often used to preserve/suspend peripheral blood mononuclear cells. Many types of cell culture medium are available, and such should be chosen to optimise the survival of the cell type for selection.

The methods of the disclosure may involve the splitting of a panel of microdroplets containing at least one cell, into a reference panel of microdroplets containing at least one cell and a test panel of microdroplets containing at least medium. It will be appreciated by those skilled in the art that some test panel microdroplets may also contain a cell, particularly if cell division has taken place in the progenitor microdroplet containing at least one cell.

In one embodiment, the microdroplets containing at least one cell have already been subjected to a splitting operation, in which a reference panel of cells containing at least one microdroplet has been created. This results in the preservation of a clonal copy of the cell, such that if stimulating the cell in the test panel during any subsequent step may result in damaging the cell, the cell from the reference panel or microdroplets may still be selected. In particular this damage may arise from the process of lysing the cell to access genetic material or intracellular products.

Where such a split is employed to separate cells and medium, the progenitor microdroplet containing at least one cell may be cultured under conditions permitting secretions to accumulate in the medium. Such conditions may include providing in the medium agents to promote the secretion, such as stimulatory or signalling molecules (for example cytokines) or including a cell such as an antigen presenting cell that interacts with the cell to induce the formation of secretions. Alternatively, if the secretion has been genetically engineered to be produced by the cell, the engineering can be applied to make expression of the secretion conditional, such that expression is activated upon the addition of a chemical, such as lactose. Thus, the appropriate signal may be provided to initiate expression.

Once the cells have been cultured to promote secretion, the panel of microdroplets may be split. Splitting operations may be conducted using real or virtual electrowetting electrodes. In one embodiment, each microdroplet in the panel of microdroplets is split to form at least two panels of child droplets. These child droplets may be approximately the same size, or the split may be asymmetric, such that one panel of microdroplets have larger dimensions. When the microdroplets are split, one child droplet may receive at least one cell, and such a microdroplet forms the reference panel. When the microdroplets are split, one child droplet may receive the medium together with any secretions, and as such a microdroplet forms the test panel.

The presence of the secretions within the medium can be ascertained as described herein. It may be useful to employ a series of merging and splitting events on microdroplets of the test panel, such as merging one or more panels of aqueous microdroplets, such that the secretion within the test panel of microdroplets may be diluted or washed.

The microdroplets containing cells and medium may be maintained at an optimal conditions for maintenance of the cells, with conditions such as environment and temperature controlled. Maintaining the cells may involve provision of a flowing stream of immiscible carrier liquid containing one or more saturation levels of nutrients, buffers, nitrogen, oxygen and/or carbon dioxide as described in WO2020/104769.

Those skilled in the art will appreciate that a microdroplet containing at least one cell will also inherently include medium since the microdroplets are aqueous in nature.

### Cell division

The methods of the present disclosure may include a step wherein the panel of microdroplets containing at least one cell are permitted to undergo cell division. Cell division is the process by which a parent cell divides into two or more daughter cells. In this context, each daughter cell may be genetically identical to the parent cell (the cell has undergone mitosis if eukaryotic). Prokaryotes (bacteria and archaea) may undergo binary fission, where their genetic material is segregated equally into two daughter cells. These cells may be described as clones, since the genetic material should be identical.

In one embodiment, panel of microdroplets containing at least one cell are permitted to undergo clonal expansion. Clonal expansion is the process by which daughter cells arise from a parent cell, and Is generally a term which is applied to specific types of cells such as immune cells (NK cells, B and T lymphocytes for example).

For the methods of the disclosure, cell division or clonal expansion is permitted or encouraged in the panel of microdroplets. The cells may be provided with appropriate conditions to permit said cell division, including provision of appropriate culture medium within the microdroplet, addition of relevant supplementary molecules, such as nutrients, cytokines, vitamins, activators, hormones or growth factors. Those skilled in the art will be aware that the conditions required for cell division will vary depending on the cell type in question.

In one embodiment, the cells present in the panel of microdroplets containing at least one cell are an immune cell, such as a lymphocyte (NK cell, B cell or T cell). Such immune cells may require activation to trigger clonal expansion. Activation may involve signalling molecules such as specific cytokines. Activation may alternatively or additionally involve binding to specific cell surface receptors on the immune cells. Such may be bound using antibodies directed to these receptors. Alternatively, activation may be provided using appropriate cells (such as antigen presenting cells, APC).

In one embodiment, the panel of microdroplets containing at least one T cell may contain or be merged with a panel of microdroplets containing at least one T cell activator, such as anti CD3 antibodies or anti CD28 antibodies.

In one embodiment, the cells may be minimally stimulated/activated so that minimal cell division takes place within the microdroplet.

Should a cell fail to activate or undergo cell division, this lack of generation of a further copy of the cell (clone) may lead to that microdroplet failing to be selected. Thus, activation and/or capability to divide may be a characteristic that is selected for in the methods of the present disclosure.

Once the cells have been cultured to promote cell division, the panel or microdroplets may be split. Splitting operations may be conducted using real or virtual electrowetting electrodes. In one embodiment, each microdroplet in the panel of microdroplets is split to form at least two panels of child droplets. These child droplets may be approximately the same size, or the split may be asymmetric, such that one panel of microdroplets have larger dimensions. When the microdroplets are split, each child droplet may receive at least one cell. Microdroplets without at least one cell may be discarded or discounted from selection. The cells in the split microdroplets are effectively genetically identical or are clones.

In one embodiment, after cell division, the panel of microdroplets containing at least one cell is split into two panels; a reference panel of microdroplets and a panel of test microdroplets which may be further used in the methods described herein. The methods of the present disclosure permit the cells of the reference panel of microdroplets to be selected on the basis of assays and/or tests on the characteristics of the cells in the panel of microdroplets containing at least one cell, since both contain cells that are genetically identical.

The methods as described here refer to the "culturing" of cells to permit cell division. Such a technique is simply the growth of the cell within the microdroplet environment. Favourable conditions for growth may be used, such as appropriate cell culture medium, nutrients and the like as discussed herein. Those skilled in the art will be aware of cell culturing techniques that promote cell growth and division.

### Identification and tracking of microdroplets

The microdroplets of the present disclosure are capable of being manipulated appropriately on a device as described herein. The control of the microdroplet manipulation may be computer implemented. The location of the microdroplet on the device permits the identification of said microdroplet, such that microdroplets that are correlated or corresponding, such as those that originated from a splitting event of a progenitor microdroplet, are identified. This permits the selection of a microdroplet from the reference panel when a reporter entity indicates a result in the test panel. Additionally, when one of the microdroplets produced from a splitting event is recovered from the microfluidic chip and dispensed in to an external microwell plate, the original progenitor microdroplet of the splitting event (that is maintained on the chip in the reference panel) can be corresponded to the result of an off-chip assay such as DNA sequencing, PCR or mass spectroscopy conducted on the related child droplet.

In one embodiment, it is not necessary to label or barcode microdroplets of any panel.

It is possible to determine from the position of the microdroplet within the panel the identity of the microdroplet. The location and therefore identity of the microdroplet may be controlled by software.

Once a characteristic of a cell is determined within a microdroplet merged with a reporter entity microdroplet, it is possible to correlate that result with the corresponding reference panel microdroplet, which relates to the same cell.

Software control of the assaying steps above allows for the sequence of operations to be adapted and modified according to the precise requirements for a particular cell type or reporter assay. For example in the case of slow-secreting cells the incubation period before a reporter panel droplet is merged can be adjusted. Software algorithms can be implemented to make the assaying steps responsive to time-dependent events occurring on the chip. In the case of cell division, image recognition software can be used to count the number of cells with in a microdroplet, and trigger a process of splitting the droplet once the cell number has reached a particular level. In some embodiments the microdroplets are droplets of fluid contained within microstructured traps such as miniature valve structures within a microfluidic chip or fluid-retaining pens within a sealed chamber structure. In some embodiments the process of splitting a droplet is carried out through application of mechanical structure or deformation of the microstructure. In some embodiments the droplets are split using acoustic manipulation.

In some embodiments the process of merging microdroplets is conducted by causing them to collide with each other using mechanical actuation, electrophoresis, acoustic waves or centrifugal force.

### Methods

The present disclosure relates to methods permitting the selection of cells, notably methods of selecting cells that permits the further use of the cells once the selection is complete.

In one embodiment, the method is as follows:
According to one aspect of the disclosure, there is provided a method for selecting cells in an EWOD or oEWOD device, the method comprising:
i. providing a test panel of microdroplets, comprising at least a medium or a medium and at least one cell;
ii. providing at least one reporter panel of microdroplets, containing one or more reporter entities;
iii. merging microdroplets of the test panel with microdroplets of the at least one reporter panel to form a panel of merged assay microdroplets; and
iv. monitoring the panel of assay microdroplets using a detection system capable of detecting a change in said reporter entity based upon the presence of at least one characteristic, and
v. selecting a subset of microdroplets from a panel on the basis of the change in the reporter entity, said subset containing the selected cells;

wherein the test panel of microdroplets comprising at least medium is created by splitting a panel of microdroplets containing at least one cell and a medium, further creating a reference panel of microdroplets containing at least one cell;
and further wherein the test panel of microdroplets comprising at least one cell are split into at least two panels of microdroplets before or after any of steps (i) to (v), said panels being: a test panel of microdroplets comprising at least medium or medium and at least one cell, suitable for merging with microdroplets of a reporter entity panel; and a reference panel of microdroplets containing at least one cell.

The cells may therefore be selected based upon one or more characteristics.

Alternatively, there is provided a method for selecting cells in an EWOD or oEWOD device, the method comprising:
i. providing a panel of microdroplets, comprising one or more cells and a medium;
ii. splitting said panel of microdroplets into at least two panels of microdroplets, at least one reference panel of microdroplets comprising one or more cells and at least one test panel of microdroplets comprising at least said medium;
iii. providing at least one reporter panel of microdroplets, containing one or more reporter entities;
iv. merging microdroplets of the test panel with microdroplets of the at least one reporter panel to form a panel of merged assay microdroplets; and
v. monitoring the panel of assay microdroplets using a detection system capable of detecting a change in said reporter entity based upon the presence of at least one characteristic, and
vi. selecting a subset of microdroplets from the reference panel on the basis of the change in the reporter entity in the corresponding assay microdroplet panel, said subset containing the selected cells.

According to another aspect of the disclosure, there is provided a method of selecting cells using an EWOD or oEWOD device comprising the steps of:
i) providing a panel of microdroplets, comprising at least one cell and medium;
ii) providing at least one panel of reporter microdroplets, comprising one or more reporter entities;
iii) merging microdroplets of the panel comprising at least one cell with microdroplets of the at least one reporter panel to form a merged panel of microdroplets;
iv) monitoring the merged panel of microdroplets using a detection system capable of detecting a change in said reporter entity based upon one or more characteristics of the cell and selecting a subset of microdroplets on the basis of said change in a reporter entity to form a selected panel of microdroplets comprising a cell;
v) taking the selected panel of microdroplets comprising a cell and repeating steps (ii) to (iv) thereon one or more times, in which one or more different reporter entities are used in step (ii);
vi) selecting cells based upon the final panel of microdroplets selected in step (v);
wherein prior to step (iii) and/or step (v) the panel of microdroplets comprising at least one cell are split into at least two panels of microdroplets, , said panels being:
a. a test panel of microdroplets, comprising at least medium, suitable for merging with microdroplets of a reporter entity panel; and
b. a reference panel of microdroplets, comprising at least one cell.

The following features may apply to any method described herein:
The microdroplets of any panel may be subjected to multiple merging and splitting operations, with the aim of diluting the contents of the microdroplet, or to "wash" the contents of the microdroplet. Such operations include the merging of the panel of microdroplets with one or more panels of microdroplets containing medium, buffer or a suitable aqueous solution and splitting the panel of merged microdroplet into at least two panels of child microdroplets. This step may be successively performed to further dilute the contents.

In one embodiment, microdroplets in the various panels are merged pairwise; such that a pair (two) microdroplets are merged to form a new microdroplet. This pairwise step may be performed more than once, such that a plurality of microdroplets are combined to form a merged microdroplet. Thus, the step of merging the microdroplets may ultimately involve 2, 3, 4, 5, 6, 7, 8, 9, 10 or more microdroplets. Each may be merged sequentially or substantially at the same time.

When sets of microdroplets are merged, effectively simultaneously, one microdroplet from each panel is merged to form a new merged microdroplet panel. The set of microdroplets for this merging operation may contain 2, 3, 4, 5, 6, 7, 8, 9, 10 or more microdroplets, each from a separate panel.

Where a plurality of panels of microdroplets containing at least one reporter entity is provided, each panel may comprise microdroplets containing at least one reporter entity. Each reporter entity may be used to determine the presence or absence of a particular cell characteristic. Alternatively, a single panel of microdroplets containing a plurality of reporter entities may be employed in a merging step.

In one embodiment, the step (ii) involves using reporter entities that are to determine cell surface characteristics of the cells, such as the presence or absence of specific cell markers or molecules. The test panel of microdroplets may then be merged with a further reporter panel of microdroplets; may then involve using reporter entities that are to determine cell:cell interaction characteristics, such as the ability to activate or kill another cell type.

Alternatively, in a different embodiment these steps are reversed such as the cells are first characterised using reporter entities that are to determine cell:cell interaction characteristics, such as the ability to activate or kill another cell type and secondly characterised using reporter entities that are to determine cell surface characteristics of the cells, such as the presence or absence of specific cell markers.

If multiple steps of merging a test panel of microdroplets with a reporter panel of microdroplets are used, it is possible that a splitting step may be employed before during or after such multiple steps, such that a reference panel of microdroplets containing a cell is constructed at any appropriate point in the method. The reference panel may therefore represent a subset of the cells originally provided.

In an embodiment, prior to merging a panel of microdroplets containing at least one cells with a panel of reporter entities containing at least one reporter entity, the microdroplets are split into at least two panels of microdroplets, such that a reference panel of microdroplets may be retained which comprises at least one cell. These cells are then not contacted with reporter entities which may be deleterious to the cell, or prevent further use of the cells in therapy and the like. The other constructed panel of microdroplets (the "test panel") may contain medium or medium and at least one cell. Prior to the splitting step, the cells may be cultured in conditions permitting either cell division or cell secretion.

In a particular embodiment, the method comprises:
A method of selecting immune cells such as T cells transfected with a nucleic acid encoding a CAR using an EWOD or oEWOD device comprising the steps of:
i) providing a panel of microdroplets, comprising at least one immune cell transfected with a nucleic acid encoding a CAR;
ii) providing at least one panel of reporter microdroplets, comprising one or more reporter entities which are specific for cell surface markers;
iii) merging microdroplets of the panel comprising at least one cell with microdroplets of the reporter panel to form a merged panel of microdroplets;
iv) monitoring the merged panel of microdroplets using a detection system capable of detecting a change in said reporter entity based upon the presence of one or more cell surface markers and selecting a subset of microdroplets on the basis of said change in a reporter entity to form a selected panel of microdroplets comprising at least one cell;
v) taking the selected panel of microdroplets comprising at least one cell and optionally repeating steps (ii) to (iv) thereon one or more times, in which one or more different reporter entities are used in step (ii) and comprise a cell;
vi) selecting cells based upon the final panel of microdroplets selected in step (iv) or (v);
wherein prior to step (iii) and/or step (v) the panel of microdroplets comprising at least one cell are cultured to permit cell division and subsequently split into at least two panels of microdroplets comprising at least one cell, said panels being:
a. a test panel of microdroplets comprising at least one cell; and
b. a reference panel of microdroplets comprising at least one cell.

The immune cells may be T cells. Suitable markers that can be detected for T cells include any one or more of the following:
CD4, CD8, CD19, scFv domain markers (for specificity and/or affinity screening), B-cell markers (or markers for other unwanted contaminant cells) and/or differentiation markers (to choose relevant sub-panels).

Cells that do not divide, activate or possess the necessary cell killing properties (T cells) may be discarded.

In a different embodiment, the method provides:
A method of selecting genetically modified haematopoietic stem cells using an EWOD or oEWOD device comprising the steps of:
i) providing a panel of microdroplets, comprising at least one genetically modified haematopoietic stem cell;
ii) providing at least one panel of reporter microdroplets, comprising one or more reporter entities;
iii) merging microdroplets of the panel comprising at least one cell with microdroplets of the reporter panel to form a merged panel of microdroplets;
iv) monitoring the merged panel of microdroplets using a detection system capable of detecting a change in said reporter entity based upon one or more characteristics of the cell and selecting a subset of microdroplets on the basis of said change in a reporter entity to form a selected panel of microdroplets comprising at least one cell;
v) taking the selected panel of microdroplets comprising at least one cell and optionally repeating steps (ii) to (iv) thereon one or more times, in which one or more different reporter entities are used in step (ii);
vi) selecting cells based upon the final panel of microdroplets selected in step (iv) or (v);
wherein prior to step (iii) and/or step (v) the panel of microdroplets comprising at least one cell are cultured to permit cell division and subsequently split into at least two panels of microdroplets comprising at least one cell, said panels being:
a. a panel of microdroplets, comprising at least one cell, suitable for merging with microdroplets of a reporter panel; and
b. a reference panel of microdroplets comprising at least one cell.

In a different embodiment, the method provides:
A method of selecting cells based upon production of immunoglobulins in an EWOD or oEWOD device, said method comprising:
i. providing a test panel of microdroplets, comprising at least a medium or a medium and an least one immune cell;
ii. providing at least one reporter panel of microdroplets, containing one or more reporter entities;
iii. merging microdroplets of the test panel with microdroplets of the at least one reporter panel to form a panel of merged assay microdroplets; and
iv. monitoring the panel of assay microdroplets using a detection system capable of detecting a change in said reporter entity based upon the presence of at least one immunoglobulin, and
v. selecting a subset of microdroplets from a panel on the basis of the change in the reporter entity, said subset containing the selected cells;

wherein the test panel of microdroplets comprising at least medium is created by splitting a panel of microdroplets containing at least one cell and a medium, further creating a reference panel of microdroplets containing at least one cell;
and/or wherein the test panel of microdroplets comprising at least one cell are split into at least two panels of microdroplets before or after any of steps (i) to (v), said panels being: a test panel of microdroplets comprising at least medium or medium and at least one cell, suitable for merging with microdroplets of a reporter entity panel; and a reference panel of microdroplets containing at least one cell.

In a different embodiment, the method provides:
A method of selecting genetically engineered cells based upon production of pharmaceutical drugs in an EWOD or oEWOD device, said method comprising:
i. providing a test panel of microdroplets, comprising at least a medium or a medium and an least one genetically engineered cell;
ii. providing at least one reporter panel of microdroplets, containing one or more reporter entities;
iii. merging microdroplets of the test panel with microdroplets of the at least one reporter panel to form a panel of merged assay microdroplets; and
iv. monitoring the panel of assay microdroplets using a detection system capable of detecting a change in said reporter entity based upon the presence of at least one pharmaceutical drug, and
v. selecting a subset of microdroplets from a panel on the basis of the change in the reporter entity, said subset containing the selected cells;

wherein the test panel of microdroplets comprising at least medium is created by splitting a panel of microdroplets containing at least one cell and a medium, further creating a reference panel of microdroplets containing at least one cell;
and/or wherein the test panel of microdroplets comprising at least one cell are split into at least two panels of microdroplets before or after any of steps (i) to (v), said panels being: a test panel of microdroplets comprising at least medium or medium and at least one cell, suitable for merging with microdroplets of a reporter entity panel; and a reference panel of microdroplets containing at least one cell.

The following features may apply to any method described herein:
The manipulation of the microdroplets within any of the panels is effected using real or virtual electrowetting electrodes. When microdroplets are merged, this involves the combination of a plurality of separate microdroplets to form a single microdroplet.

Optionally this method may include a further step of permitting differentiation on the device prior to any merging step. The reporter entities may determine activity of the transgene introduced via genetic engineering, or may look for specific cell surface markers, cell products or cell secretions.

In an alternative embodiment, the method is to select particular genetically modified cells on the basis of expression of the genetically added transgene, where the same may be used to produce a product (such as a chemical or protein).

A method of selecting genetically engineered cells using an EWOD or oEWOD device comprising the steps of:
i) providing a panel of microdroplets, comprising at least one genetically engineered cell;
ii) providing at least one panel of reporter microdroplets, comprising one or more reporter entities;
iii) merging microdroplets of the panel comprising at least one cell with microdroplets of the reporter panel to form a merged panel of microdroplets;
iv) monitoring the merged panel of microdroplets using a detection system capable of detecting a change in said reporter entity based upon one or more characteristics of the cell and selecting a subset of microdroplets on the basis of said change in a reporter entity to form a selected panel of microdroplets comprising at least one cell;
v) taking the selected panel of microdroplets comprising at least one cell and repeating steps (ii) to (iv) thereon one or more times, in which one or more different reporter entities are used in step (ii);
vi) selecting cells based upon the final panel of microdroplets selected in step (v);
wherein prior to step (iii) and/or step (v) the panel of microdroplets comprising at least one cell are cultured to permit cell division and subsequently split into at least two panels of microdroplets comprising at least one cell, said panels being:
a. a test panel of microdroplets, comprising at least one cell; and
b. a reference panel of microdroplets comprising at least one cell.

Optionally, the method may further include the step of lysing the cells in the microdroplets of the panel prior to merging with a reporter panel of microdroplets. Such a step is generally undertaken after the reference panel of microdroplets each containing at least one cell has been established.

The cells may be any type of cell suitable for genetic modification, such as mammalian, insect, plant, bacterial or fungal.

Any combination of the methods disclosed herein is contemplated, and the method steps and order in which the method is performed, will depend upon the nature of the cell under selection and the characteristics which are desired.

Microdroplets may also be referred to as droplets.

### Examples

Certain aspects and embodiments of the invention will now be illustrated by way of example and with reference to the figures described above. The methods described herein were performed on a proprietary microfluidic device (Lightcast Discovery LTD, UK).

### Example 1 - Exemplar CAR-T Workflow

This workflow targets a particular window in the CAR-T cell manufacturing process starting with the genetic modification of T-cells and ending before bulk expansion and reinfusion of selected T-cells into a patient (as depicted in fig. 1).

Fundamentally, a two-part screen is used for the selection of CAR-T cells involving: 1) a multi-reporter cell-surface marker assay; and 2) activation assessment, and these steps can be performed in either order.

Patient blood is collected and patient-derived T-cells (unmodified) are isolated. These T-cells are emulsified into microdroplets by conventional means and loaded onto the microfluidic platform. The T-cells are then transduced - this step involves merging a panel of said microdroplets, each containing a T-cell, with a panel of microdroplets, each containing a vector, pairwise, to form a first panel of merged microdroplets. The vector comprises a nucleic acid that is operably linked to a promotor, wherein the nucleic acid sequence encodes a chimeric antigen receptor. After transduction, the T-cells proceed to the first screening step. Empty or multi-occupancy microdroplets are discarded where necessary (fig. 2).

The multi-element reporter assay may involve the use of a detection system to analyse multiple markers on the transduced T-cells, microdroplets are then selected on this basis. The reporter scheme can, for example, include the following markers: CD4/CD8, CD19, scFv domain markers (for specificity and/or affinity screening), B-cell markers (or markers for other unwanted cells) and/or differentiation markers (to choose relevant sub-panels). Analysing multiple markers on the T-cells involves the steps of merging microdroplets containing the transduced T-cells with microdroplets containing reporters (e.g. primary antibodies and fluorescent dye-conjugated detection antibodies). Sub-optimal cells are removed here at the earliest possible stage, before expansion. This gives the best possible input for later expansion and profiling steps. From here, the selected microdroplets then proceed to dosimetry and profiling stages (as depicted on fig. 2).

A reference panel of CAR-T cells may be constructed by splitting a panel of microdroplets containing at least one CAR-T cell after the cell has been activated and allowed to divide.

Cells of the selected microdroplets are expanded on-chip, assessed for viability, and for activation and exhaustion behaviour. The activity screens could, for example, include screening for anti-tumour activity and potency. Detailed methods for assessing anti-tumour cell activity are described in Example 3. According to the results of these assays, unwanted microdroplets are discarded and a final panel of microdroplets is selected, which may be a subset of the reference panel. The on-chip activity assays will, for example, involve merging microdroplets containing doses of T-cells with microdroplets containing tumour cell(s) or tumoroid(s) at various T-cell/tumour cell ratios and measuring the cancer-cell killing activity of the T-cells. Dose efficacy and safety profiles can be assessed at this stage. The genomes of these T-cells can be sequenced and single cell genomic data can be acquired here (fig. 3).

T-cells of the final selected sub panel of microdroplets can be dispensed from the device into a well plate for bulk expansion and later reinfusion into the patient (fig. 3). This method permits the best possible consistency and viability of the selected cells and enables quantification of efficacy and safety profile information.

### Example 2 - Immuno-oncology Workflow

Chinese hamster ovary (CHO) cells are modified to produce an immuno-therapeutic drug (e.g. a TCR) and then emulsified into microdroplets and loaded onto the microfluidic platform. Empty or multi-occupancy microdroplets are discarded. The remaining microdroplets containing single CHO cells are incubated on-chip to promote the production of the immunotherapeutic drug. The CHO containing droplets are then split to obtain multiple doses of the drug produced by each cell. T cells and target tumour cells are separately emulsified and loaded into arrays onto the microfluidic platform, adjusting the cell occupancy of each microdroplet as desired. The T cell and tumour cell arrays are then merged. A second merge operation is used to add a dose of immunotherapeutic drug, tracking which CHO cell each dose came from. The resulting assay is incubated and T-cell killing behaviour is monitored using the detection system by detecting caspase 3/7 fluorescence, a fluorescent marker of apoptosis. CHO cells that produced effective doses of the test drug can be dispensed from the device into a well plate (fig. 4).

### Example 3 - Pan T-cell Killing Assay

### Preparation Protocol

A tumour model cell line (passage 59+1) cultured in EMEM (10% FCS, 1% glutamine, 1% penicillin/streptomycin) at 80% confluence is then incubated in droplet media (RPMI 1640 (10% FCS, 1% glutamine, 1% penicillin/streptomycin)) at a density of 3e6/mL supplemented with 1 µM caspase 3/7, 50 µM Hoechst 33342 and 0.1 nM TCR drug for 2 hours at 5% CO₂ and 37°C. Meanwhile, human donor derived Pan T Cells are thawed and washed in PBS and then incubated in 1 µM Deep Red for 30 minutes at 5% CO₂ and 37°C before incubating in droplet media (RPMI 1640) at a density of 2e6/mL. Both cell suspensions are then emulsified into microdroplets (fig. 5).

### Droplet Panel

The microdroplets containing Pan T-cell(s) are then sorted into a first panel and the microdroplets containing tumour cell(s) are sorted into a second panel. The two panels are then merged pairwise to generate microdroplets containing both T-cell(s) and tumour cell(s) at various tumour cell(s)/Pan-T cell(s) ratios. Microdroplets containing only tumour cell(s) are also included as a negative control (fig. 6). The merged microdroplets are scanned for caspase 3/7 fluorescence at t=0 hours to confirm that no tumour cells have apoptosed prematurely.

### Results

Results indicated that the 50% viability window is at approximately 28 hours which is sufficient time to see clear cancer cell killing behaviour (fig. 7). Caspase intensity is measured for each droplet over time to quantify time-dependent tumour cell apoptosis. Brightfield microscopy is used to image T-cells interacting with and serially killing tumour cells within each microdroplet (fig. 8, fig. 9).

### Example 4 - Antibody Discovery

### Cell Viability

Irrelevant hybridoma cells were stained with zombie stain, emulsified into microdroplets and loaded onto the microfluidic platform. The cells were then assessed for viability by imaging the microdroplets (10x lens, Zombie green/GFP (excitation: 457/50, emission: 520/28): exposure time 0.54 s, 100% lamp power, BF: exposure time 0.1 s). Heat killed irrelevant hybridoma cells were used as a positive control. Cell viability remained at 100% (33 cells) for up to 5.3 hours in droplets and 3.6 hours in the device. Moreover, cell viability remained at >90% for up to 20.3 hours in droplets and 18.6 hours in device (28 cells, 1 droplet with 5 cells not retained throughout the assay). Therefore excellent viability was demonstrated during and beyond the assay window. This can be partly attributed to a gas resupply mechanism which is used in this system and contributes towards the sustained viability observed (fig. 10).

### Negative Controls

Microdroplets containing hybridoma cells and microdroplets containing reporters (antigen conjugated beads and secondary detection antibodies) were loaded onto the lightcast platform and merged pairwise to generate microdroplets containing: 1 bead/2 cells, 1 bead/1 cell, beads only and cells only (fig. 11). No fluorescence was detected from negative control microdroplets containing only hybridoma cells or reporters. Cells were also stained with Zombie Green stain - no cell death was detected during the course of the experiment. The assay was run for up to 16 hours and 20 minutes. Fluorescent aggregates started appearing at t = 3 hours and 20 minutes in microdroplets containing hybridomas and reporters.

### Bulk Assay

Hybridoma cells were emulsified into microdroplets and loaded onto the microfluidic platform. Hybridoma media containing reporters (e.g. antigen conjugated beads and AF488-conjugated secondary antibodies) were also emulsified into microdroplets and loaded onto the device. Microdroplets containing hybridoma cells were sorted into a first panel and microdroplets containing reporters were sorted into a second panel. The first and second panels were merged pairwise to generate microdroplets containing 0.5 M/mL beads, 0.5 M/mL cells and 200 nM AF488-conjugated secondary antibody in hybridoma media and incubated for 2 hours at 37°C. Bead fluorescence was imaged (BB1, 10x objective, 2 s exp. time) for secreted anti-target antibody.

Further microdroplets were generated containing 2 M/mL beads, 200 nM AF488-conjugated secondary antibody and 10-100 nM [free anti-target antibody (Anti-mouse AF488)] in hybridoma media incubated for 4.5 hours at room temperature. Bead fluorescence was imaged (BB1, 10x objective, 2 s exp. time) across the concentration range of free anti-target antibody tested to generate a curve showing filtered fluorescence (a.u.) against anti-target antibody concentration (fig. 12).

Microdroplets containing irrelevant hybridoma cells were consistently dark. In contrast, fluorescence close to maximum level observed for free anti-target antibody was measured for secreting target hybridoma cells (fig. 12). The detection system scans for increased AF488 fluorescence intensity of the bead.

### Irrelevant/Target Hybridomas

Cell Tracker Deep Red fluorescence stain was applied to the irrelevant hybridomas before emulsification, and used to identify them on the microfluidic device. In this case irrelevant hybridomas are defined as hybridoma cells which secrete a protein that should not be detected by the reporter molecules. Microdroplets containing hybridoma cells and microdroplets containing reporters were loaded onto the Microfluidic platform and merged pairwise to generate microdroplets containing a range of different irrelevant or target hybridoma cell(s)/bead(s) ratios. Negative control microdroplets containing only reporters were also prepared. Negative controls and beads incubated with cells identified as irrelevant via Deep Red did not appear to light up for the duration of the experiment (16 hours). Beads incubated with target hybridomas (hybridomas that produce anti-target antigen primary antibody) showed a clear AF488 signal at 2 hours and 20 minutes post merging (fig. 13).

### Multiplexed Spiking Assay

The multiplexed spiking assay was used to separate a mixed population of microdroplets, each containing either irrelevant or target hybridoma cell(s). This assay required a large number of microdroplets, all of which were imaged at 10X resolution or better. Two separate cultures of hybridoma cells (Target and Irrelevant) were grown and then harvested in to media. Target and irrelevant hybridoma cell(s) were then emulsified separately into microdroplets and loaded onto the microfluidic platform. The microdroplets, each containing either target or irrelevant hybridoma cell(s) were sorted into a first panel. Medium containing reporters (target antigen conjugated beads and [AF488-conjugated anti-mouse secondary antibody] 200 nM) was also emulsified into microdroplets and sorted into a second panel. The first and second panels were merged pairwise to generate a panel of merged microdroplets. Filtered fluorescence ((exc. 457/50nm)/a.u.) on the beads was measured for each merged microdroplet over time (fig. 13) in a spiking run to generate anti-target antibody secretion curves for each hybridoma cell line. The AF488 signal from irrelevant hybridoma cell lines was indistinguishable from negative controls. All target hybridomas showed a positive signal. Aggregates began to form in droplets after approximately 4 hours and started to impair analysis after 9 hours.

Single cell secretion data was also collected and single-cell secretion curves were generated showing time dependent antibody secretion from single cells. Beads were loaded at 128 nM and bead fluorescence was measured over 7 hours counting from bead - antibody merge. The results were replicated in multiple runs. To collect single cell data, multi-occupancy hybridoma cell microdroplets were discarded prior to merging first and second panels.

### Multiplexed Beads

Imaging was tested on the microfluidic platform in bulk in hybridoma media. Microdroplets, each containing a high concentration of beads (SolR1, R3 and R5 beads) were loaded onto the device. Imaging was tested at different magnifications (2x, 4x and 10x) to determine the optimum parameters for imaging whilst achieving good separation between all intensity bands (R1, R3 and R5) enabling beads to be properly resolved for intensity quantification. Good separation between all intensity bands was achieved at 10x and fair separation was achieved at 4x (fig. 14).

Colour coded beads (solR1, R3 and R5) were conjugated with the following antigens respectively: cyno target antigen, off-target antigen and human target antigen. Cell culture medium comprising the antigen conjugated beads and anti-mouse AF488 detection antibody was then emulsified into microdroplets and loaded onto the Lightcast platform. Microdroplets each containing single target or irrelevant hybridoma cells were also provided and split generating a panel of microdroplets, each containing secreted product (e.g. primary antibody) and a reference panel of microdroplets, each containing corresponding hybridoma cell(s), tracking which hybridoma cell each dose of secreted antibody came from. The microdroplets containing reporters (beads and detection antibody) were then merged with the microdroplets containing secreted antibody pairwise. The characteristics of the merged microdroplets and control microdroplets were monitored using the Lightcast detection system capable of both decoding the bead intensity band (and therefore identifying the antigen) and quantifying coincident AF488 fluorescence (enables quantification of anti-target primary antibody). Negative control microdroplets containing no cells (reporters only) and merged microdroplets containing irrelevant hybridoma cells remained dark confirming the absence of primary antibodies. Positive control microdroplets containing reporters and free target antibody [25 nM] confirmed binding of free target antibody to both target antigens (R1 and R5). Microdroplets containing secreted antibody also bound both target antigens (fig. 15).

### Antibody Discovery Workflow

B cells are emulsified into microdroplets and loaded onto the microfluidic platform. Empty or multi-occupancy microdroplets are discarded. The remaining microdroplets containing single B cells are merged with microdroplets containing cell culture media pairwise for added volume to facilitate subsequent splitting steps. Afterwards, the merged microdroplets are incubated on-chip to promote the production of monoclonal antibody and then split into daughter droplets to obtain multiple doses of the antibody produced by each cell. The microdroplets containing B-cells are then sorted into a reference panel whilst the microdroplets containing doses of antibody are sorted into a separate panel and merged pairwise with a reporter panel of microdroplets containing antigen-conjugated beads and detection antibodies. An optical detection system is used to determine positive hits in the merged reporter-mAb panel.

Microdroplets of the reference panel containing B-cells corresponding to said positive hits are dispensed into a well plate and expanded (fig. 16).

### Example 5 - Bacterial Workflow

Bacterial cells are emulsified into microdroplets and loaded onto the Lightcast platform. Empty or multi-occupancy microdroplets are discarded. The remaining microdroplets containing single bacterial cells are merged with microdroplets containing cell culture media pairwise for added volume to facilitate subsequent splitting step(s). The merged microdroplets are expanded and then split into clonal colonies. One set of microdroplets containing clonal bacterial cells is sorted into a reference panel whilst a further set of microdroplets containing clonal bacterial cells or a set of microdroplets containing excretions of said bacterial cells is sorted into an assay panel of microdroplets. The assay panel of microdroplets is merged pairwise with a reporter panel of microdroplets. At any stage the bacterial cells may be lysed to release their contents if the assay components are internal to the bacterial membrane. An optical detection system is used to determine positive hits in the merged reporter-bacterial cell panel. Microdroplets of the reference panel containing bacterial cells corresponding to said positive hits are dispensed into a well plate and expanded (fig. 17).

### Example 6 - Brightfield imaging of merging and/or splitting microdroplets

Figures 19A to 19C show brightfield images of an exemplary merge operation. Pairs of droplets are merged in parallel throughout the device. Figure 19A shows pairs of microdroplets selected for a merging operation. Figure 19B shows paired microdroplets moving into close proximity ready for merging. Figure 19C shows the merged microdroplets.

Figures 20A to 20C show brightfield images of an exemplary splitting operation. Microdroplets are split in parallel into pairs of two daughter microdroplets. Figure 20A shows droplets in an array awaiting a split operation. Figure 20B shows stretched microdroplets midway through a splitting operation. Figure 20C shows microdroplets split into pairs of daughter droplets. Historic information can be retained through operations to give full information on progenitor droplets.

Referring to Figure 21, there is shown a brightfield image of *S. cerevisiae* bio-particles (heat inactivated yeast) encapsulated in droplets at single cell occupancy.

### Example 7 - Cells encapsulated with reporter beads within droplets in an emulsion of cell media in oil

Referring to Figure 22, there is shown images of cytokine secreting cells which have been co-encapsulated with cytokine reporter beads within droplets in an emulsion of cell media in oil. In the presence of secreted cytokine, the reporter beads fluoresce via a sandwich ELISA mechanism using a fluorescently tagged antibody. As shown in Figure 22A, the brightfield image shows the distribution of cells and beads within the microdroplets. Fluorescence images as shown in Figure 22B, indicates which reporter bead gives a positive response in the presence of secreted cytokines. Figure 22C shows the position of all cells with intact membrane integrity. Fluorescence images shown in Figure 22D indicates the position of all beads, and Figure 22E shows the dead cells as indicated by propidium iodide staining.

Referring to Figure 24, there is shown images of antibody secreting cells, which are co-encapsulated with reporter beads within microdroplets in an emulsion of cell media in oil. In the presence of secreted antibody, the reporter beads fluoresce via a sandwich ELISA mechanism using a fluorescently tagged antibody. The brightfield image in Figure 24A shows the distribution of cells and beads within the microdroplets. Fluorescence images shown Figure 24B illustrates which reporter bead(s) gives a positive response in the presence of secreted antibody. Figure 24C shows the position of all cells with intact membrane integrity, and Figure 24D shows dead cells.

### Example 8 - Microdroplet modular design of operations

Figure 23 outlines the modular design of operations which can be flexibly combined in full or in part in any order to build a unique workflow. Programmable decisions can be implemented at any point throughout the workflow, which can divert from the original order and/or apply operations to only a selection of microdroplets based on navigation of decision tree logic. As such, the assay can be dynamically reconfigured. Exemplary modules are shown including but not limited to loading and/or sorting microdroplets, merging microdroplets, incubating, assaying, splitting, and exporting. Examples of microdroplet sorting may include, but is not limited to, sorting based on size, content and/or occupancy. Merging droplets can allow the delivery of reagents and/or reporters, and enables the controlled introduction of cells to facilitate cell-cell interactions. Splitting droplets can enable materials to be recovered to downstream analysis or, in the case of cell recovery, further expansion.

The workflow as illustrated in Figure 25 exemplifies a variety of detection modes available for monitoring cell secretions or cell-cell interactions. The detection can be, but is not limited to, fluorescence detection, luminescence, FRET or brightfield detection. Intracellular imaging can reveal signal within part of a cell or cell container, examples include the nucleus, Golgi apparatus and/or mitochondria.

### Example 9 - Synthetic biology Workflow, mammalian cell line development and/or CRISPR screening

Figure 26 outlines an exemplary workflow suitable for synthetic biology, mammalian cell line development and/or CRISPR screening, but not exclusive to these screens. Cells engineered to produce a product of interest are loaded onto the platform and contained within droplets, which can be sorted for occupancy to select for single cells. Cells can be incubated to enable a period of proliferation on the platform. Monitoring proliferation can be used to select for robust clones. Microdroplets can be optionally split to control droplet size, or to collect either product or cells for downstream analysis whilst retaining a population for alternative operations. Retention of a population is particularly useful for carrying out assay steps that may result in degradation of the cells on a daughter microdroplet whilst retaining live cells for further testing in a second daughter microdroplet. Merging allows control of droplet size and delivery of components to cell-droplets, for example media to extend cell lifetime and/or a reporter. A reporter can facilitate titre readout via various detection modes to assess cell productivity. Microdroplets can be dispensed off the platform and recovered. Recovery of cells may enable further expansion or downstream analysis. As an example only, the recovery of product can enable an assessment of product quality. The order of operations can be changed freely. Microdroplets can be dispensed based on assessments carried out during the screening procedure e.g. top clones with high productivity and good proliferation.

### Example 10 - Sorting microdroplet operation workflow

Referring to Figure 27, there is provided an exemplary workflow following an example sequence of modular operations and indicates potential points throughout the workflow where microdroplets can be optionally sorted. Sorting can be based on numerous factors such as droplet size, content, occupancy, assay results or a combination thereof. Brightfield, fluorescence, luminescence, FRET are some examples of detection modalities for sorting, where signal can be within the microdroplet, localised to a reporter or intracellular.

### Example 11 - Brightfield imaging of cells encapsulated within microdroplets

Referring to Figures 28A to 28D, there is provided a plurality of images of cells encapsulated within the microdroplets, incubated in the presence of a dye for intracellular Calcium. Figure 28A shows brightfield images of droplets and indicates the position and number of cells within each droplet. Figure 28B shows a fluorescence image indicating active Calcium metabolism. Figure 28C shows a fluorescence image of nuclear staining of the cells, and Figure 28D shows dead cells indicated by propidium iodide staining.

Various further aspects and embodiments of the present invention will be apparent to those skilled in the art in view of the present disclosure.
"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

It will further be appreciated by those skilled in the art that although the invention has been described by way of example with reference to several embodiments, it is not limited to the disclosed embodiments and that alternative embodiments could be constructed without departing from the scope of the invention as defined in the appended claims.

## Claims

1. A method for selecting cells and/or part(s) of a cell(s) based on at least one characteristic in an EWOD (electrowetting on dielectric) or oEWOD (optically mediated electrowetting on dielectric) device, the method comprising:
i. providing a test panel of microdroplets, comprising at least a medium or a medium and at least one cell;
ii. providing at least one reporter panel of microdroplets, containing one or more reporter entities;
iii. merging microdroplets of the test panel with microdroplets of the at least one reporter panel to form a panel of merged assay microdroplets; and
iv. monitoring the panel of assay microdroplets using a detection system capable of detecting a change in said reporter entity based upon the presence of at least one characteristic, and
v. selecting a subset of microdroplets from a panel on the basis of the change in the reporter entity, said subset containing the selected cells;
wherein the test panel of microdroplets comprising at least medium is created by splitting a panel of microdroplets containing at least one cell and a medium, further creating a reference panel of microdroplets containing at least one cell;
and/or wherein the test panel of microdroplets comprising at least one cell are split into at least two panels of microdroplets before or after any of steps (i) to (v), said panels being: a test panel of microdroplets comprising at least medium or medium and at least one cell, suitable for merging with microdroplets of a reporter entity panel; and a reference panel of microdroplets containing at least one cell.

2. The method as claimed in claim 1, wherein said characteristic of a cell is selected from any one or more of: the presence of one or more cell surface molecules; the presence of one or more cellular activities; cellular morphology; the presence of one or more cellular secretions; and/or the presence of one or more intracellular products, optionally wherein a characteristic of said cell is viability, cell activation and/or cell killing ability.

3. The method as claimed in claim 1 or claim 2, wherein prior to splitting the panel of microdroplets containing at least one cell, said cell is cultured under conditions permitting cell division, optionally:
i) wherein said conditions include merging the panel of microdroplets including at least one cell with a panel of microdroplets containing medium;
ii) wherein the reference panel and the test panel of microdroplets are split such that the reference panel of microdroplets contains at least one clonal copy of a cell corresponding to a cell contained in the test panel of microdroplets, and/or,
iii) wherein said test panel of microdroplets containing at least one cell is merged with a panel of microdroplets containing an agent for lysing said cells.

4. The method according to any preceding claim, wherein cells in any panel of microdroplets containing at least one cell are inspected for their morphological characteristics and said morphological characteristics may be used to select a subset of cells, optionally wherein said morphological characteristics include size, shape, adhesion state, cell membrane state and/or the presence of intracellular features.

5. The method according to any one of the preceding claims, wherein prior to step (ii) the panel of microdroplets containing at least one cell may be subjected to at least one assay to determine a characteristic of the cell, said assay being performed by merging said panel of microdroplets containing at least one cell with at least one panel of microdroplets containing at least one reporter entity and determining a change in the at least one reporter entity based upon the presence of said characteristic.

6. The method according to any preceding claim, wherein after step (iv), at least one panel of aqueous microdroplets may be merged with the merged assay panel of microdroplets, and subsequently the merged panel of microdroplets are split, optionally wherein at any stage in the method, unwanted or empty microdroplets are discarded.

7. The method according to any preceding claim, wherein cells of the selected panel of microdroplets are dispensed from the device, optionally wherein after dispensing the selected panel of microdroplets :
i) the cells contained therein are treated to expand the population and/or to culture clone(s), and/or
ii) the cells and/or non-cellular entities such as secretions, metabolites contained therein are subjected to further analysis, including, but not limited to any one or more of the following:
I. mass spectrometry;
II. surface plasmon resonance;
III. high - performance liquid chromatography; and/or
IV. genetic analysis, including nucleic acid sequencing and PCR amplification.

8. The method according to any preceding claim, wherein the microdroplets of the test panel correspond to the microdroplets of the reference panel, such that the cells retained in the reference panel may be selected on the basis of a change in said reporter entity in the test panel of microdroplets.

9. The method according to any preceding claim, wherein the cell is genetically engineered, optionally wherein the cell may be genetically engineered in advance of step (i), comprising the steps of:
a. providing a panel of microdroplets, each comprising at least one cell;
b. providing a panel of modification microdroplets; each comprising one or more genetic elements capable of modifying a cell;
c. merging microdroplets containing a cell with microdroplets containing one or more genetic elements to form a panel of microdroplets, each containing a genetically engineered cell.

10. The method according to any preceding claim, wherein a characteristics of said cells is that they are capable of making any one or more of the following products as intracellular products, cell surface molecules and/or secretions which may be detected using the reporter entity:
i. a protein, including a glycoprotein or a lipoprotein;
ii. a chemical;
iii. a polymer;
iv. a nucleic acid;
v. a compound.

11. The method according to any preceding claim, wherein the at least one reporter entity is any one or more of the following entities:
i. an antibody;
ii. an antigen;
iii. a receptor;
iv. a substrate;
v. an enzyme;
vi. a ligand;
vii. a nucleic acid;
viii. a cell;
ix. a part of a cell;
x. an extracellular vesicle;
xi. a liposome;
xii. a polymer;
xiii. a chemical;
xiv. a drug;
xv. a FRET reporter;
xvi. a chemiluminescent material;
xvii. a sample of tissue;
xviii. a virus or bacteriophage;
xix. a cytokine; and /or
xx. a protein.

12. The method according to any preceding claim, wherein said reporter entity is labelled for direct detection of a change, and/ or a second labelled entity is also included, which can detect and report the change in the reporter entity, optionally wherein said label is visible, luminescent, fluorescent, phosphorescent, protein complementation such as split fluorescence, split luminescence or fluorogenic ligands.

13. The method according to any preceding claim, wherein said cells are selected from any one of the following types:
i. immune cell;
ii. bacteria; optionally wherein said bacterial cell is genetically engineered to produce a drug;
iii. fungal cell;
iv. insect cell;
v. pluripotent cell;
vi. cancer cell;
vii. a hybridoma cell-fusion; optionally wherein said hybridoma cell-fusion cells are capable of secretion of immunoglobulins;
viii. cell line;
ix. plant cell
x. artificial cell;
xi. microcell
xii. a part(s) of a cell(s);
xiii. an extracellular vesicle; or
xiv. a liposome;
optionally wherein said cell is capable of producing an immunotherapeutic drug.

14. The method according to any preceding claim, wherein said cells are lymphocytes, optionally:
a) wherein said cells are B lymphocytes and are capable of secretion of immunoglobulins;
b) wherein said reporter entity is a reporter cell, optionally wherein desired immunoglobulins are capable of binding to the reporter cell and causing a change, or
c) wherein the one or more reporter entities are selected from:
i. antigen-conjugated beads and fluorescent-dye conjugated secondary antibodies;
ii. secondary-antibody-conjugated beads and fluorescent-dye conjugated antigens; or
iii. antigen conjugated to a carrier surface and fluorescent-dye conjugated secondary antibodies beads
and wherein monitoring the characteristics of the immunoglobulin comprises assaying for antigen binding activity.

15. The method according to any one of claims 1 to 14, optionally wherein said cells:
i) have been genetically engineered to express either a cell surface receptor or T cell engaging molecules;
ii) are immune cells and said cell surface receptor is a chimeric antigen receptor, optionally wherein the method is to select CAR-T cells, and/or,
iii) have been isolated from a patient.

16. The method as claimed in any preceding claim, wherein the microdroplets are surrounded by an immiscible liquid, optionally wherein more than one reporter entity is present in each microdroplet of the reporter panel, optionally wherein step (v) is a detection of a multiplex assay.

## Patentansprüche

1. Verfahren zum Auswählen von Zellen und/oder eines Teils/von Teilen einer Zelle/von Zellen auf der Grundlage von mindestens einer Eigenschaft in einer EWOD(Elektrobenetzung auf Dielektrikum)- oder oEWOD(optisch vermittelte Elekrobenetzung auf Dielektrikum)-Vorrichtung, wobei das Verfahren Folgendes umfasst:
i. Bereitstellen eines Testpanels von Mikrotröpfchen, die mindestens ein Medium oder ein Medium und mindestens eine Zelle umfassen;
ii. Bereitstellen mindestens eines Reporterpanels von Mikrotröpfchen, die eine oder mehrere Reportereinheiten enthalten;
iii. Zusammenführen von Mikrotröpfchen des Testpanels mit Mikrotröpfchen des mindestens einen Reporterpanels, um ein Panel zusammengeführter Untersuchungsmikrotröpfchen zu bilden; und
iv. Überwachen des Panels von Untersuchungsmikrotröpfchen unter Verwendung eines Nachweissystems, das in der Lage ist, eine Änderung in der Reportereinheit basierend auf dem Vorhandensein mindestens einer Eigenschaft nachzuweisen, und
v. Auswählen einer Teilmenge von Mikrotröpfchen aus einem Panel auf der Grundlage der Änderung in der Reportereinheit, wobei die Teilmenge die ausgewählten Zellen enthält;
wobei das Testpanel von Mikrotröpfchen, die mindestens ein Medium umfassen, durch Aufteilen eines Panels von Mikrotröpfchen, die mindestens eine Zelle und ein Medium enthalten, erzeugt wird, wodurch ferner ein Referenzpanel von Mikrotröpfchen erzeugt wird, die mindestens eine Zelle enthalten;
und/oder wobei das Testpanel von Mikrotröpfchen, die mindestens eine Zelle umfassen, vor oder nach einem der Schritte (i) bis (v) in mindestens zwei Panels von Mikrotröpfchen aufgeteilt wird, wobei die Panels Folgendes sind: ein Testpanel von Mikrotröpfchen, die mindestens ein Medium oder ein Medium und mindestens eine Zelle umfassen und zum Zusammenführen mit Mikrotröpfchen eines Reportereinheitenpanels geeignet sind; und ein Referenzpanel von Mikrotröpfchen, die mindestens eine Zelle enthalten.

2. Verfahren nach Anspruch 1, wobei die Eigenschaft einer Zelle aus einem oder mehreren von Folgenden ausgewählt wird: Vorhandensein eines oder mehrerer Zelloberflächenmoleküle; Vorhandensein einer oder mehrerer Zellaktivitäten; Zellmorphologie; Vorhandensein eines oder mehrerer Zellsekrete; und/oder Vorhandensein eines oder mehrerer intrazellulärer Produkte, wobei eine Eigenschaft der Zelle optional Lebensfähigkeit, Zellaktivierungs- und/oder Zellabtötungsfähigkeit ist.

3. Verfahren nach Anspruch 1 oder Anspruch 2, wobei vor dem Aufteilen des Panels von Mikrotröpfchen, die mindestens eine Zelle enthalten, die Zelle unter Bedingungen kultiviert wird, die eine Zellteilung ermöglichen, optional:
i) wobei die Bedingungen ein Zusammenführen des Panels von Mikrotröpfchen, die mindestens eine Zelle beinhalten, mit einem Panel von Mikrotröpfchen, die ein Medium enthalten, beinhalten;
ii) wobei das Referenzpanel und das Testpanel von Mikrotröpfchen so aufgeteilt werden, dass das Referenzpanel von Mikrotröpfchen mindestens eine Klonkopie einer Zelle enthält, die einer in dem Testpanel von Mikrotröpfchen enthaltenen Zelle entspricht, und/oder
iii) wobei das Testpanel von Mikrotröpfchen, die mindestens eine Zelle enthalten, mit einem Panel von Mikrotröpfchen zusammengeführt wird, die ein Mittel zum Lysieren der Zellen enthalten.

4. Verfahren nach einem vorhergehenden Anspruch, wobei Zellen in einem beliebigen Panel von Mikrotröpfchen, die mindestens eine Zelle enthalten, auf ihre morphologischen Eigenschaften geprüft werden und die morphologischen Eigenschaften verwendet werden können, um eine Teilmenge von Zellen auszuwählen, wobei die morphologischen Eigenschaften optional Größe, Form, Adhäsionszustand, Zellmembranzustand und/oder das Vorhandensein intrazellulärer Merkmale beinhalten.

5. Verfahren nach einem der vorhergehenden Ansprüche, wobei vor Schritt (ii) das Panel von Mikrotröpfchen, die mindestens eine Zelle enthalten, mindestens einer Untersuchung unterzogen werden kann, um eine Eigenschaft der Zelle zu bestimmen, wobei die Untersuchung durchgeführt wird, indem das Panel von Mikrotröpfchen, die mindestens eine Zelle enthalten, mit mindestens einem Panel von Mikrotröpfchen, die mindestens eine Reportereinheit enthalten, zusammengeführt wird und eine Änderung in der mindestens einen Reportereinheit auf der Grundlage des Vorhandenseins der Eigenschaft bestimmt wird.

6. Verfahren nach einem vorhergehenden Anspruch, wobei nach Schritt (iv) mindestens ein Panel von wässrigen Mikrotröpfchen mit dem zusammengeführten Untersuchungspanel von Mikrotröpfchen zusammengeführt werden kann und das zusammengeführte Panel von Mikrotröpfchen anschließend aufgeteilt wird, wobei optional in jeder Phase des Verfahrens unerwünschte oder leere Mikrotröpfchen verworfen werden.

7. Verfahren nach einem vorhergehenden Anspruch, wobei Zellen des ausgewählten Panels von Mikrotröpfchen aus der Vorrichtung abgegeben werden, wobei optional nach dem Abgeben des ausgewählten Panels von Mikrotröpfchen:
i) die darin enthaltenen Zellen behandelt werden, um die Population zu vergrößern und/oder einen Klon(e) zu kultivieren, und/oder
ii) die Zellen und/oder nichtzellulären Einheiten wie Sekrete und Metaboliten, die darin enthalten sind, einer weiteren Analysen unterzogen werden, einschließlich, jedoch nicht beschränkt auf eine oder mehrere von Folgenden:
I. Massenspektrometrie;
II. Oberflächenplasmonenresonanz;
III. Hochleistungsflüssigkeitschromatographie; und/oder
IV. genetische Analyse, einschließlich Nukleinsäuresequenzierung und PCR-Amplifikation.

8. Verfahren nach einem vorhergehenden Anspruch, wobei die Mikrotröpfchen des Testpanels den Mikrotröpfchen des Referenzpanel entsprechen, sodass die in dem Referenzpanel zurückgehaltenen Zellen auf der Grundlage einer Änderung in der Reportereinheit in dem Testpanel von Mikrotröpfchen ausgewählt werden können.

9. Verfahren nach einem vorhergehenden Anspruch, wobei die Zelle gentechnisch verändert wird, wobei die Zelle optional vor Schritt (i) gentechnisch verändert werden kann, was die folgenden Schritte umfasst:
a. Bereitstellen eines Panel von Mikrotröpfchen, von denen jedes mindestens eine Zelle umfasst;
b. Bereitstellen eines Panels von Modifikations-Mikrotröpfchen; von denen jedes ein oder mehrere genetische Elemente umfasst, die in der Lage sind, eine Zelle zu modifizieren;
c. Zusammenführen von Mikrotröpfchen, die eine Zelle enthalten, mit Mikrotröpfchen, die ein oder mehrere genetische Elemente enthalten, um ein Panel von Mikrotröpfchen zu bilden, von denen jedes eine gentechnisch veränderte Zelle enthält.

10. Verfahren nach einem vorhergehenden Anspruch, wobei eine Eigenschaft der Zellen darin besteht, dass sie in der Lage sind, eines oder mehrere der folgenden Produkte als intrazelluläre Produkte, Zelloberflächenmoleküle und/oder Sekrete herzustellen, die unter Verwendung der Reportereinheit nachgewiesen werden können:
i. ein Protein, einschließlich eines Glykoproteins oder eines Lipoproteins;
ii. eine Chemikalie;
iii. ein Polymer;
iv. eine Nukleinsäure;
v. eine Verbindung.

11. Verfahren nach einem vorhergehenden Anspruch, wobei die mindestens eine Reportereinheit eine oder mehrere der folgenden Einheiten ist:
i. ein Antikörper;
ii. ein Antigen;
iii. ein Rezeptor;
iv. ein Substrat;
v. ein Enzym;
vi. ein Ligand;
vii. eine Nukleinsäure;
viii. eine Zelle;
ix. ein Teil einer Zelle;
x. ein extrazelluläres Vesikel;
xi. ein Liposom;
xii. ein Polymer;
xiii. eine Chemikalie;
xiv. ein Arzneimittel;
xv. ein FRET-Reporter;
xvi. ein chemilumineszierendes Material;
xvii. eine Gewebeprobe;
xviii. ein Virus oder Bakteriophage;
xix. ein Zytokin; und/oder
xx. ein Protein.

12. Verfahren nach einem vorhergehenden Anspruch, wobei die Reportereinheit zum direkten Nachweis einer Änderung markiert ist und/oder eine zweite markierte Einheit ebenfalls beinhaltet ist, die die Änderung in der Reportereinheit nachweisen und melden kann, wobei die Markierung optional sichtbar, lumineszierend, fluoreszierend, phosphoreszierend, eine Proteinkomplementierung wie beispielsweise Spaltfluoreszenz, Spaltlumineszenz oder fluorogene Liganden ist.

13. Verfahren nach einem vorhergehenden Anspruch, wobei die Zellen aus einem der folgenden Typen ausgewählt werden:
i. Immunzelle;
ii. Bakterien; wobei die Bakterienzelle optional gentechnisch so verändert ist, dass sie ein Arzneimittel produziert;
iii. Pilzzelle;
iv. Insektenzelle;
v. pluripotente Zelle;
vi. Krebszelle;
vii. eine Hybridomzellfusion; wobei die Hybridomzellfusionszellen optional zur Sekretion von Immunglobulinen fähig sind;
viii. Zelllinie;
ix. Pflanzenzelle
x. künstliche Zelle;
xi. Mikrozelle
xii. ein Teil (Teile) einer Zelle (von Zellen);
xiii. ein extrazelluläres Vesikel; oder
xiv. ein Liposom;
wobei die Zelle optional in der Lage ist, ein immuntherapeutisches Arzneimittel zu produzieren.

14. Verfahren nach einem vorhergehenden Anspruch, wobei die Zellen Lymphozyten sind, optional:
a) wobei die Zellen B-Lymphozyten sind und zur Sekretion von Immunglobulinen fähig sind;
b) wobei die Reportereinheit eine Reporterzelle ist, wobei optional gewünschte Immunglobuline in der Lage sind, an die Reporterzelle zu binden und eine Änderung zu verursachen, oder
c) wobei die eine oder mehreren Reportereinheiten aus Folgenden ausgewählt sind:
i. Antigen-konjugierte Beads und Fluoreszenzfarbstoff-konjugierte Sekundärantikörper;
ii. Sekundärantikörper-konjugierte Beads und Fluoreszenzfarbstoff-konjugierte Antigene; oder
iii. Antigen, das an eine Trägeroberfläche konjugiert ist, und Beads mit Fluoreszenzfarbstoffkonjugierten sekundären Antikörpern
und wobei ein Überwachen der Eigenschaften des Immunglobulins Untersuchen auf Antigen-Bindungsaktivität umfasst.

15. Verfahren nach einem der Ansprüche 1 bis 14, wobei die Zellen optional:
i) genetisch verändert wurden, um entweder einen Zelloberflächenrezeptor oder T-Zellen-Verbindungsmoleküle zu exprimieren;
ii) Immunzellen sind und der Zelloberflächenrezeptor ein chimärer Antigenrezeptor ist, wobei das Verfahren optional dazu dient, CAR-T-Zellen auszuwählen, und/oder
iii) aus einem Patienten isoliert wurden.

16. Verfahren nach einem vorhergehenden Anspruch, wobei die Mikrotröpfchen durch eine nicht mischbare Flüssigkeit umgeben sind, wobei optional mehr als eine Reportereinheit in jedem Mikrotröpfchen des Reporterpanels vorhanden ist, wobei optional Schritt (v) ein Nachweis einer Multiplex-Untersuchung ist.

## Revendications

1. Méthode permettant la sélection de cellules et/ou d'une ou de parties d'une ou de cellules sur la base d'au moins une caractéristique dans un dispositif d'EWOD (électromouillage sur diélectrique) ou d'oEWODZ (électromouillage à médiation optique sur diélectrique), la méthode comprenant :
i. la fourniture d'un panel de test de microgouttelettes, comprenant au moins un milieu ou un milieu et au moins une cellule ;
ii. la fourniture d'au moins un panel rapporteur de microgouttelettes, contenant une ou plusieurs entités rapporteuses ;
iii. la fusion de microgouttelettes du panel de test avec des microgouttelettes dudit au moins un panel rapporteur pour former un panel de microgouttelettes de dosage fusionnées ; et
iv. la surveillance du panel de microgouttelettes de dosage à l'aide d'un système de détection pouvant détecter un changement dans ladite entité rapporteuse sur la base de la présence d'au moins une caractéristique, et
v. la sélection d'un sous-ensemble de microgouttelettes à partir d'un panel sur la base du changement dans l'entité rapporteuse, ledit sous-ensemble contenant les cellules sélectionnées ;
ledit panel de test de microgouttelettes comprenant au moins un milieu étant créé en scindant un panel de microgouttelettes contenant au moins une cellule et un milieu, en créant en outre un panel de référence de microgouttelettes contenant au moins une cellule ;
et/ou ledit panel de test de microgouttelettes comprenant au moins une cellule étant scindé en au moins deux panels de microgouttelettes avant ou après l'une quelconque des étapes (i) à (v), lesdits panels étant : un panel de test de microgouttelettes comprenant au moins un milieu ou un milieu et au moins une cellule, adapté à la fusion avec des microgouttelettes d'un panel d'entités rapporteuses ; et un panel de référence de microgouttelettes contenant au moins une cellule.

2. Méthode selon la revendication 1, ladite caractéristique d'une cellule étant choisie parmi une ou plusieurs parmi : la présence d'une ou plusieurs molécules de surface cellulaire ; la présence d'une ou plusieurs activités cellulaires ; la morphologie cellulaire ; la présence d'une ou plusieurs sécrétions cellulaires ; et/ou la présence d'un ou plusieurs produits intracellulaires, éventuellement une caractéristique de ladite cellule étant la viabilité, l'activation des cellules et/ou la capacité de destruction des cellules.

3. Méthode selon la revendication 1 ou la revendication 2, avant la scission du panel de microgouttelettes contenant au moins une cellule, ladite cellule étant cultivée dans des conditions permettant la division cellulaire, éventuellement :
i) lesdites conditions comprenant la fusion du panel de microgouttelettes comprenant au moins une cellule avec un panel de microgouttelettes contenant un milieu ;
ii) ledit panel de référence et ledit panel de test de microgouttelettes étant scindés de sorte que le panel de référence de microgouttelettes contienne au moins une copie clonale d'une cellule correspondant à une cellule contenue dans le panel de test de microgouttelettes, et/ou,
iii) ledit panel de test de microgouttelettes contenant au moins une cellule étant fusionné avec un panel de microgouttelettes contenant un agent permettant la lyse desdites cellules.

4. Méthode selon l'une quelconque des revendications précédentes, lesdites cellules dans n'importe quel panel de microgouttelettes contenant au moins une cellule étant examinées pour leurs caractéristiques morphologiques et lesdites caractéristiques morphologiques pouvant être utilisées pour sélectionner un sous-ensemble de cellules, éventuellement lesdites caractéristiques morphologiques comprenant la taille, la forme, l'état d'adhérence, l'état de la membrane cellulaire et/ou la présence d'éléments intracellulaires.

5. Méthode selon l'une quelconque des revendications précédentes, avant l'étape (ii), ledit panel de microgouttelettes contenant au moins une cellule pouvant être soumis à au moins un dosage pour déterminer une caractéristique de la cellule, ledit dosage étant effectuée en fusionnant ledit panel de microgouttelettes contenant au moins une cellule avec au moins un panel de microgouttelettes contenant au moins une entité rapporteuse et en déterminant un changement dans ladite au moins une entité rapporteuse sur la base de la présence de ladite caractéristique.

6. Méthode selon l'une quelconque des revendications précédentes, après l'étape (iv), au moins un panel de microgouttelettes aqueuses pouvant être fusionné avec le panel de dosage fusionné de microgouttelettes, et ensuite ledit panel fusionné de microgouttelettes étant scindé, éventuellement, à n'importe quelle étape de la méthode, lesdites microgouttelettes indésirables ou vides étant éliminées.

7. Méthode selon l'une quelconque des revendications précédentes, lesdites cellules du panel sélectionné de microgouttelettes étant distribuées par le dispositif, éventuellement après la distribution du panel sélectionné de microgouttelettes :
i) lesdites cellules qu'il contient étant traitées pour accroître la population et/ou pour cultiver un ou des clones, et/ou
ii) lesdites cellules et/ou entités non cellulaires telles que les sécrétions, les métabolites qu'elles contiennent étant soumises à des analyses approfondies, y compris mais de façon non limitative, à une quelconque ou plusieurs des suivantes :
I. la spectrométrie de masse ;
II. la résonance plasmonique de surface ;
III. la chromatographie liquide haute performance ; et/ou
IV. l'analyse génétique, y compris le séquençage des acides nucléiques et l'amplification par PCR.

8. Méthode selon l'une quelconque des revendications précédentes, lesdites microgouttelettes du panel de test correspondant aux microgouttelettes du panel de référence, de sorte que les cellules retenues dans le panel de référence puissent être sélectionnées sur la base d'un changement de ladite entité rapporteuse dans le panel de test de microgouttelettes.

9. Méthode selon l'une quelconque des revendications précédentes, ladite cellule étant génétiquement modifiée, éventuellement ladite cellule pouvant être génétiquement modifiée avant l'étape (i), comprenant les étapes de :
a. fourniture d'un panel de microgouttelettes, chacune comprenant au moins une cellule ;
b. fourniture d'un panel de microgouttelettes de modification ; chacune comprenant un ou plusieurs éléments génétiques capables de modifier une cellule ;
c. fusion de microgouttelettes contenant une cellule avec des microgouttelettes contenant un ou plusieurs éléments génétiques pour former un panel de microgouttelettes, chacune contenant une cellule génétiquement modifiée.

10. Méthode selon l'une quelconque des revendications précédentes, une caractéristique desdites cellules étant qu'elles sont capables de fabriquer l'un quelconque ou plusieurs des produits suivants en tant que produits intracellulaires, molécules de surface cellulaire et/ou sécrétions qui peuvent être détectés à l'aide de l'entité rapporteuse :
i. une protéine, y compris une glycoprotéine ou une lipoprotéine ;
ii. un produit chimique ;
iii. un polymère ;
iv. un acide nucléique ;
v. un composé.

11. Méthode selon l'une quelconque des revendications précédentes, ladite au moins une entité rapporteuse étant l'une quelconque ou plusieurs des entités suivantes :
i. un anticorps ;
ii. un antigène ;
iii. un récepteur ;
iv. un substrat ;
v. une enzyme ;
vi. un ligand ;
vii. un acide nucléique ;
viii. une cellule ;
ix. une partie d'une cellule ;
x. une vésicule extracellulaire ;
xi. un liposome ;
xii. un polymère ;
xiii. un produit chimique ;
xiv. un médicament ;
xv. un rapporteur de FRET ;
xvi. un matériau chimioluminescent ;
xvii. un échantillon de tissu ;
xviii. un virus ou un bactériophage ;
xix. une cytokine ; et/ou
xx. une protéine.

12. Méthode selon l'une quelconque des revendications précédentes, ladite entité rapporteuse étant marquée pour la détection directe d'un changement, et/ou une seconde entité marquée étant également comprise, qui peut détecter et rapporter le changement dans l'entité rapporteuse, éventuellement ledit marqueur étant visible, luminescent, fluorescent, phosphorescent, une complémentation de protéines telle que la fluorescence divisée, la luminescence divisée ou des ligands fluorogènes.

13. Méthode selon l'une quelconque des revendications précédentes, lesdites cellules étant choisies parmi l'un quelconque des types suivants :
i. une cellule immunitaire ;
ii. des bactéries ; éventuellement ladite cellule bactérienne étant génétiquement modifiée pour produire un médicament ;
iii. une cellule fongique ;
iv. une cellule d'insecte ;
v. une cellule pluripotente ;
vi. une cellule cancéreuse ;
vii. une fusion de cellules d'hybridome ; éventuellement lesdites cellules de fusion de cellules d'hybridome étant capables de sécréter des immunoglobulines ;
viii. une lignée cellulaire ;
ix. une cellule végétale
x. une cellule artificielle ;
xi. une microcellule
xii. une ou des parties d'une ou de cellules ;
xiii. une vésicule extracellulaire ; ou
xiv. un liposome ;
éventuellement ladite cellule étant capable de produire un médicament immunothérapeutique.

14. Méthode selon l'une quelconque des revendications précédentes, lesdites cellules étant des lymphocytes, éventuellement :
a) lesdites cellules étant des lymphocytes B et étant capables de sécréter des immunoglobulines ;
b) ladite entité rapporteuse étant une cellule rapporteuse, éventuellement lesdites immunoglobulines souhaitées étant capables de se lier à la cellule rapporteuse et de provoquer un changement, ou
c) lesdites une ou plusieurs entités rapporteuses étant choisies parmi :
i. des billes conjuguées à un antigène et des anticorps secondaires conjugués à un colorant fluorescent ;
ii. des billes conjuguées à un anticorps secondaire et des antigènes conjugués à un colorant fluorescent ; ou
iii. des antigènes conjugués à une surface porteuse et des billes d'anticorps secondaires conjuguées à un colorant fluorescent
et ladite surveillance des caractéristiques de l'immunoglobuline comprenant le dosage de l'activité de liaison à un antigène.

15. Méthode selon l'une quelconque des revendications 1 à 14, éventuellement lesdites cellules :
i) ayant été génétiquement modifiées pour exprimer soit un récepteur de surface cellulaire, soit des molécules engageant les lymphocytes T ;
ii) étant des cellules immunitaires et ledit récepteur de surface cellulaire étant un récepteur d'antigène chimérique, éventuellement ladite méthode étant la sélection de lymphocytes CAR-T, et/ou,
iii) ayant été isolées d'un patient.

16. Méthode selon l'une quelconque des revendications précédentes, lesdites microgouttelettes étant entourées d'un liquide non miscible, éventuellement plus d'une entité rapporteuse étant présente dans chaque microgouttelette du panel rapporteur, éventuellement ladite étape (v) étant une détection d'un dosage multiplex.
